(19) Europäisches Patentamt
European Patent Office
Office européen des brevets

(11) EP 1 275 648 A1

(12) # EUROPÄISCHE PATENTANMELDUNG

(43) Veröffentlichungstag:
**15.01.2003 Patentblatt 2003/03**

(21) Anmeldenummer: **02022332.7**

(22) Anmeldetag: **18.06.1999**

(51) Int Cl.[7]: **C07D 493/04**, C07D 417/06, C07D 413/06, C07D 277/24 // (C07D493/04, 313:00, 303:00)

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **18.06.1998 DE 19826988**

(62) Dokumentnummer(n) der früheren Anmeldung(en) nach Art. 76 EPÜ:
**99932700.0 / 1 087 975**

(71) Anmelder: **Gesellschaft für Biotechnologische Forschung mbH (GBF)**
**38124 Braunschweig (DE)**

(72) Erfinder:
- **Höfle, Gerhard, Prof. Dr.**
  **38124 Braunschweig (DE)**
- **Reichenbach, Hans, Prof. Dr.**
  **38124 Braunschweig (DE)**
- **Gerth, Klaus, Dr.**
  **38124 Braunschweig (DE)**
- **Hardt, Ingo, Dr.**
  **38124 Braunschweig (DE)**
- **Sasse, Florenz, Dr.**
  **38124 Braunschweig (DE)**
- **Steinmetz, Heinricht**
  **38124 Braunschweig (DE)**

(74) Vertreter: **Boeters, Hans Dietrich, Dr. et al**
**Patentanwälte Boeters & Bauer,**
**Bereiteranger 15**
**81541 München (DE)**

Bemerkungen:
Diese Anmeldung ist am 08 - 10 - 2002 als Teilanmeldung zu der unter INID-Kode 62 erwähnten Anmeldung eingereicht worden.

(54) **Epothilon-Nebenkomponenten**

(57) Die Erfindung betrifft Verbindungen, die durch Fermentation von DSM 6773 erhältlich sind, insbesondere Epothilone A1, A2, A8, A9, B10, C1, C2, C3, C4, C5, C6, C7, C8, C9, D1, D2, D5, G1, G2, H1, H2, I1, I2, I3 I4, I5, I6 und K und Trans-Epothilone C1 und C2.

EP 1 275 648 A1

## Beschreibung

Die Erfindung betrifft Verbindungen, die im vorliegenden Zusammenhang als Epothilon-Nebenkomponenten bezeichnet werden, und zwar Verbindungen 5 bis 13 und 16 bis 39. Diese Verbindungen lassen sich durch Fermentation von DSM 6773 gemäß DE 41 38 042.8 gewinnen.

Kenndaten der erfindungsgemäßen Verbindungen sind im folgenden zusammengestellt.

Gewinnung: Die Aufarbeitung eines Rohepothilon-Gemischs, das durch Fermentation von DSM 6773 in einem 900 Liter-Fermentator gewonnen wurde, ist schematisch Fig. 1 bis 2 zu entnehmen.

Aktivitäten: vgl. Tab. 1

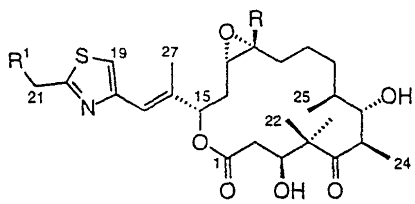

Epothilone A (**1**) $R^1 = H$; $R = H$

Epothilone B (**2**) $R^1 = H$; $R = Me$

Epothilone E (**3**) $R^1 = OH$; $R = H$

Epothilone F (**4**) $R^1 = OH$; $R = Me$

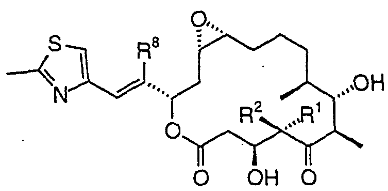

Epothilone $A_1$ (**5**) $R^1 = H$; $R^2, R^8 = Me$

Epothilone $A_2$ (**6**) $R^2 = H$; $R^1, R^8 = Me$

Epothilone $A_8$ (**7**) $R^8 = H$; $R^1, R^2 = Me$

Epothilone $A_9$ (**8**) $R^1 = CH_2OH$; $R^2, R^8 = Me$

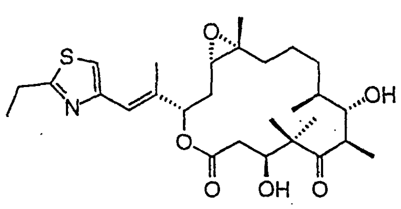

Epothilone $B_{10}$ (**9**)

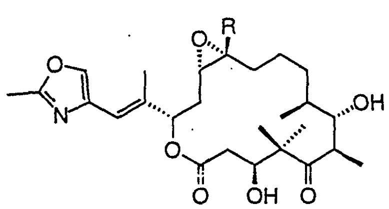

Epothilone $G_1$ (**10**) $R = H$

Epothilone $G_2$ (**11**) $R = Me$

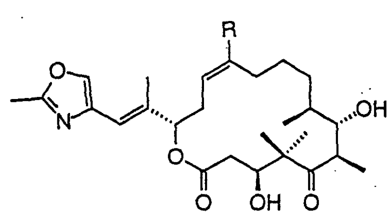

Epothilone $H_1$ (**12**) $R = H$

Epothilone $H_2$ (**13**) $R = Me$

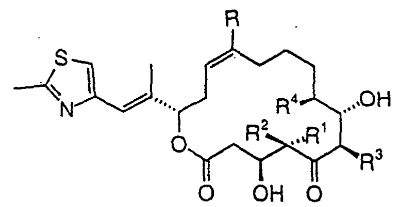

Epothilone C (**14**) $R^1, R^2, R^3, R^4 = Me$; $R = H$

Epothilone D (**15**) $R^1, R^2, R^3, R^4, R = Me$

Epothilone $C_1$ (**16**) $R^1 = H$; $R^2, R^3, R^4 = Me$; $R = H$

Epothilone $D_1$ (**17**) $R^1 = H$; $R^2, R^3, R^4 = Me$; $R = Me$

Epothilone $C_2$ (**18**) $R^2 = H$; $R^1, R^3, R^4 = Me$; $R = H$

Epothilone $D_2$ (**19**) $R^2 = H$; $R^1, R^3, R^4 = Me$; $R = Me$

Epothilone $C_3$ (**20**) $R^3 = H$; $R^1, R^2, R^4 = Me$; $R = H$

Epothilone $C_4$ (**21**) $R^4 = H$; $R^1, R^2, R^3 = Me$; $R = H$

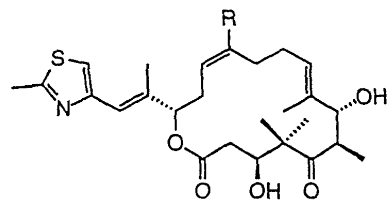

Epothilone $C_5$ (**22**) $R = H$

Epothilone $D_5$ (**23**) $R = Me$

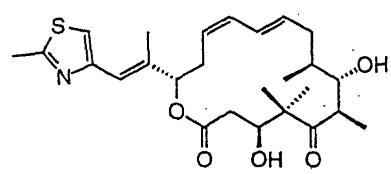

Epothilone $C_6$ (**24**)

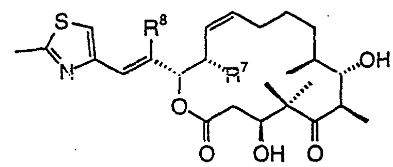

Epothilone $C_7$ (**25**) $R^7 = OH$; $R^8 = Me$

Epothilone $C_8$ (**26**) $R^8, R^7 = H$

Epothilone $C_9$ (**27**) $R^8 = CH_2OH$; $R^7 = H$

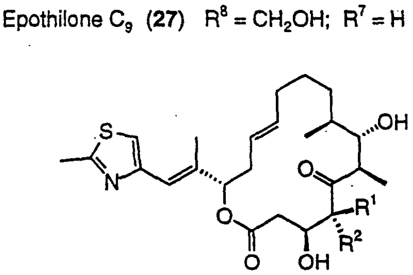

trans-Epothilone $C_1$ (**28**) $R^1 = H$; $R^2 = Me$

trans-Epothilone $C_2$ (**29**) $R^2 = H$; $R^1 = Me$

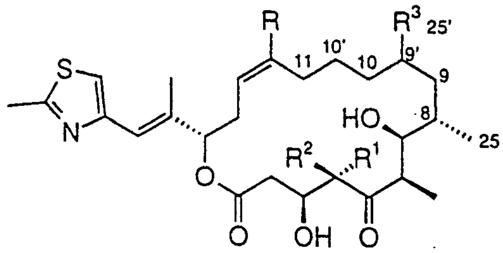

Epothilone $I_1$ (**30**) R, $R^3$ = H; $R^1$, $R^2$ = Me

Epothilone $I_2$ (**31**) R = H; $R^1$, $R^2$, $R^3$ = Me

Epothilone $I_3$ (**32**) $R^1$, $R^2$, $R^3$, R = Me

Epothilone $I_4$ (**33**) $R^2$, R = H; $R^1$, $R^3$ = Me

Epothilone $I_5$ (**34**) $R^2$ = H; $R^1$, $R^3$, R = Me

Epothilone $I_6$ (**35**) $R^1$ = H; $R^2$, $R^3$, R = Me

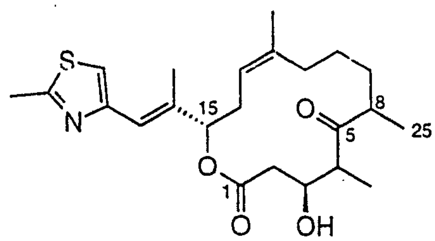

Epothilone K (**36**)

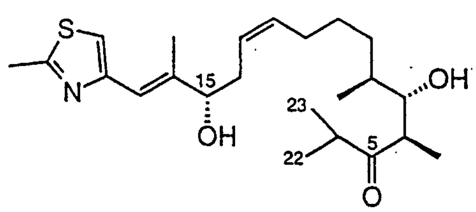

**(37)**

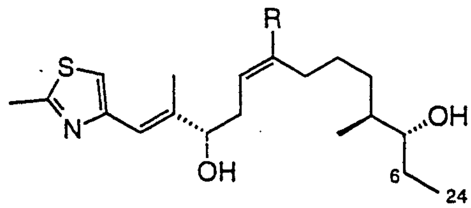

**(38)** R = H

**(39)** R = Me

**Epothilone $A_1$ (5):** colorless amorphous solid; $[\alpha]^{22}_D$ -69 (*c* 0.1, MeOH); UV (MeOH) $\lambda_{max}$ nm ($\varepsilon$) 208 (19600), 247 (13600); IR (KBr) $\nu_{max}$ 3437, 2959, 2931, 2876, 1732, 1710, 1455, 1259, 978 $cm^{-1}$; $^1H$ NMR ($CDCl_3$, 400 MHz) $\delta$ 6.95 (1H, s, H-19), 6.60 (1H, bs, H-17), 5.68 (1H, dd, *J* = 4.4, 4.0 Hz, H-15), 4.12 (1H, m, H-3), 3.71 (1H, m, H-7), 3.52 (1H, bs, 7-OH), 3.37 (1H, bd, *J* = 7.5 Hz, 3-OH), 3.21 (1H, dq, *J* = 7.7, 7.0 Hz, H-4), 3.02 (1H, ddd, *J* = 9.2, 4.5, 2.8 Hz, H-13), 2.87 (1H, ddd, *J* = 8.3, 4.5, 3.7 Hz, H-12), 2.78 (1H, dd, *J* = 16.8, 4.3 Hz, H-2a), 2.70 (3H, s, H-21), 2.66 (1H, dq, *J* = 3.9, 7.0 Hz, H-6), 2.65 (1H, dd, *J* = *16.8, 5.2* Hz, H-2b), 2.16 (1H, ddd, *J* = 15.4, 4.4, 2.8 Hz, H-14a), 2.12 (3H, bs, H-27), 1.91 (1H, ddd, *J*= 15.4, 9.2, 4.0 Hz, H-14b), 1.63 (1H, m, H-10a), 1.62 (2H, m, H-11), 1.59 (1H, m, H-9a), 1.52 (1H, m, H-10b), 1.39 (1H, m, H-8), 1.35 (1H, m, H-9b), 1.211 (3H, d, *J*= 7.0 Hz, H-23), 1.207 (3H, d, *J* = 7.0 Hz, H-24), 0.89 (3H, d, *J* = 6.9 Hz, H-25); EIMS *m/z* 479 $[M]^+$ (21), 322 (31), 306 (65), 304 (47), 168 (45), 166 (73), 164 (100), 151 (30), 140 (35); HREIMS *m/z* 479.2317 (calcd. for $C_{27}H_{41}NO_5S$, 479.2342).

**Epothilone $A_2$ (6):** colorless amorphous solid; $[\alpha]^{22}_D$ +12.0 (*c* 1.0, MeOH); UV (MeOH) $\lambda_{max}$ nm ($\varepsilon$) 210 (15100), 248 (15500); IR (KBr) $\nu_{max}$ 3438, 2963, 2929, 2875, 1734, 1706, 1458, 1262, 981 $cm^{-1}$; $^1H$ NMR ($CDCl_3$, 400 MHz) $\delta$ 6.98 (1H, s, H-19), 6.63 (1H, bs, H-17), 5.40 (1H, dd, *J* = 8.3, 3.4 Hz, H-15), 4.26 (1H, ddd, *J* = 8.5, 4.8, 4.7 Hz, H-3), 3.85 (1H, dd, *J* = 7.9, 2.6 Hz, H-7), 3.54 (1H, bs, 3-OH), 3.09 (1H, dq, *J*= 4.8, 7.0 Hz, H-4), 3.01 (1H, ddd, *J*= 8.3, 4.8, 4.6 Hz, H-13), 2.98 (1H, dq, *J*= 7.9, 7.0 Hz, H-6), 2.89 (1H, ddd, *J*= 6.7, 4.6, 4.4 Hz, H-12), 2.68 (3H, s, H-21), 2.60 (1H, dd, *J*= 15.1, 8.5 Hz, H-2a), 2.52 (1H, bs, 7-OH), 2.50 (1H, dd, *J*= 15.1, 4.7 Hz, H-2b), 2.18 (1H, ddd, *J*= 15.0, 4.8,3.4 Hz, H-14a), 2.11 (3H, d, *J*= 1.3 Hz, H-27), 1.82 (1H, ddd, *J*= 15.0, 8.3, 8.1 Hz, H-14b), 1.63 (1H, m, H-8), 1.61 (2H, m, H-11a and H-10a), 1.46 (1H, m, H-11b), 1.39 (2H, m, H-9), 1.31 (1H, m, H-10b), 1.22 (3H, d, *J*= 7.0 Hz, H-24), 1.15 (3H, d, *J* = 7.0 Hz, H-22), 1.01 (3H, d, *J*= 6.9 Hz, H-25); $^{13}C$ NMR ($CDCl_3$, 100 MHz) $\delta$ 216.2 (s, C-5), 170.1 (s, C-1), 164.9 (s, C-20), 152.0 (s, C-18), 137.0 (s, C-16), 120.3 (d, C-17), 116.5 (d, C-19), 76.7 (d, C-15), 75.6 (d, C-7), 69.1 (d, C-3), 57.1 (d, C-12), 54.3 (d, C-13), 50.3 (d, C-4), 49.6 (d, C-6), 39.4 (t, C-2), 35.5 (d, C-8), 32.2 (t, C-14), 29.6 (t, C-9), 27.6 (t, C-11), 23.9 (t, C-10), 19.2 (q, C-21), 18.0 (q, C-25), 15.6 (q, C-27), 13.9 (q, C-24), 12.4 (q, C-22); EIMS *m/z* 479 $[M]^+$ (18), 322 (38), 306 (78), 304 (59), 168 (48), 166 (96), 164 (100), 151 (33), 140 (38); HREIMS *m/z* 479.2318 (calcd. for $C_{27}H_{41}NO_5S$, 479.2342).

**Epothilone $A_8$ (7):** colorless amorphous solid; $[\alpha]^{22}_D$ -76.2 (*c* 1.0, MeOH); UV (MeOH) $\lambda_{max}$ nm ($\varepsilon$) 210 (15300), 248 (15500); IR (KBr) $\nu_{max}$ 3440, 2967, 2932, 2876, 1736, 1691, 1467, 1252, 979 $cm^{-1}$; $^1H$ NMR ($CDCl_3$, 400 MHz) $\delta$ 6.95 (1H, s, H-19), 6.64 (1H, dd, *J*= 15.6, 0.9 Hz, H-17), 6.52 (1H, dd, *J*= 15.6, 6.6 Hz, H-16), 5.68 (1H, dddd, *J* = 7.8, 6.6, 3.2, 0.9 Hz, H-15), 4.11 (1H, ddd, *J*= 10.1, 6.6, 3.5 Hz, H-3), 3.78 (1H, ddd, *J*= 5.2, 3.2, 3.2 Hz, H-7), 3.66 (1H, d, *J* = 6.6 Hz, 3-OH), 3.23 (1H, dq, *J* = 5.2, 6.9 Hz, H-6), 3.08 (1H, ddd, *J*= 7.3, 5.5 ,4.1 Hz, H-13), 2.90 (1H, ddd, *J* = 6.6, 4.6, 4.1 Hz, H-12), 2.69 (3H, s, H-21), 2.52 (1H, dd, *J*= 14.7, 10.1 Hz, H-2a), 2.44 (1H, bd, *J* = 3.2 Hz, 7-OH), 2.41 (1H, dd, *J*= 14.7, 3.5 Hz, H-2b), 2.10 (1H, ddd, *J* = 15.0, 5.5, 3.2 Hz, H-14a), 1.90 (1H, ddd, *J* = 15.0, 7.8, 7.3 Hz, H-14b), 1.71 (1H, m, H-8), 1.65 (1H, m, H-11a), 1.50 (1H, m, H-10a), 1.47 (1H, m, H-11b), 1.40 (2H, m, H-9), 1.39 (1H, m, H-10b), 1.33 (3H, s, H-23), 1.16 (3H, d, *J* = 6.9 Hz, H-24), 1.08 (3H, s, H-22), 0.98 (3H, d, *J* = 7.0 Hz, H-25); $^{13}C$ NMR ($CDCl_3$, 75 MHz) $\delta$ 220.3 (s, C-5), 170.7 (s, C-1), 166.5 (s, C-20), 152.2 (s, C-18), 128.4 (d, C-16), 125.9 (d, C-17), 116.4 (d, C-19), 75.0 (d, C-7), 73.6 (d, C-3), 72.7 (d, C-15), 57.3 (d, C-12), 54.1 (d, C-13), 52.6 (s, C-4), 43.8 (d, C-6), 38.9 (t, C-2), 36.3 (d, C-8), 32.5 (t, C-14), 30.3 (t, C-9), 26.7 (t, C-11), 24.0 (t, C-10), 21.3 (q, C-23), 21.0 (q, C-22), 19.3 (q, C-21), 17.1 (q, C-25), 14.5 (q, C-24); **EIMS *m/z* 479 $[M]^+$ XXX;** HRDCIMS *m/z* 480.2401 (calcd. for $C_{25}H_{38}NO_6S$, 480.2401).

**Epothilone $A_9$ (8)**: colorless amorphous solid; $[\alpha]^{22}_D$ -37.6 (*c* 0.5, MeOH); UV (MeOH) $\lambda_{max}$ nm ($\varepsilon$) 211

(15500), 253 (14100); IR (KBr) $\nu_{max}$ 3423, 2965, 2932, 2877, 1736, 1690, 1463, 1249, 1014, 979 $cm^{-1}$; $^{1}H$ NMR ($CDCl_3$, 400 MHz) δ 7.10 (1H, s, H-19), 6.72 (1H, dd, *J*= 10.7, 4.3 Hz, 27-OH), 6.60 (1H, bs, H-17), 5.69 (1H, dd, *J*= 11.6, 2.0 Hz, H-15), 5.59 (1H, d, *J*= 6.6 Hz, 3-OH), 4.49 (1H, ddd, *J*= 12.9, 4.3, 1.2 Hz, H-27a), 4.27 (1H, ddd, *J*= 11.6, 6.6, 2.9 Hz, H-3), 4.11 (1H, ddd, *J*= 12.9, 10.7, 1.0 Hz, H-27b), 3.71 (1H, ddd, *J*=4.8, 3.0, 2.8 Hz, H-7), 3.17 (1H, dq, *J*= 3.0, 6.8 Hz, H-6), 3.04 (1H, ddd, *J* = 9.7, 3.6, 2.2 Hz, H-13), 2.93 (1H, bs, 7-OH), 2.91 (1H, ddd, *J*= 9.7, 3.6, 2.7 Hz, H-12), 2.72 (3H, s, H-21), 2.48 (1H, dd, *J* = 14.2, 11.6 Hz, H-2a), 2.11 (1H, dd, *J* = 14.2, 2.9 Hz, H-2b), 2.03 (1H, ddd, *J*= 14.7, 2.2, 2.0 Hz; H-14a), 1.86 (1H, m, H-11a), 1.85 (1H, m, H-14b), 1.79 (1H, m, H-8), 1.52 (1H, m, H-10a), 1.37 (3H, m, H-9 and H-10b), 1.37 (3H, s, H-23), 1.36 (1H, in, H-11b), 1.19 (3H, d, *J*= 6.8 Hz, H-24), 1.02 (3H, d, *J* = 7.1 Hz, H-25), 1.00 (3H, s, H-22); $^{13}C$ NMR ($CDCl_3$, 75 MHz) δ 220.5 (s, C-5), 170.2 (s, C-1), 167.5 (s, C-20), 150.7 (s, C-18), 138.9 (s, C-16), 125.2 (d, C-17), 119.5 (d, C-19), 76.7 (d, C-15), 73.4 (d, C-7), 70.4 (d, C-3), 57.7 (d, C-12), 57.2 (t, C-27), 55.3 (d, C-13), 54.2 (s, C-4), 41.3 (d, C-6), 40.7 (t, C-2), 37.5 (d, C-8), 31.8 (t, C-14), 31.2 (t, C-9), 28.0 (t, C-11), 23.7 (q, C-23), 23.2 (t, C-10), 19.2 (q, C-21), 16.8 (q, C-22), 15.8 (q, C-25), 13.5 (q, C-24); EIMS *m/z* 509 $[M]^+$ (9), 491 (4), 322 (28), 321 (25), 180 (45), 167 (40), 166 (100), 165 (49), 154 (47), 138 (33); HREIMS *m/z* 509.2467 (calcd. for $C_{26}H_{39}NO_7S$, 509.2447).

**Epothilone $B_{10}$ (9):** colorless amorphous solid; $[\alpha]^{22}$ **-27 (*c* 0.15, MeOH); UV (MeOH) $\lambda_{max}$ nm (ε) 212 (15800), 247 (12500);** IR (KBr) $\nu_{max}$ 3434, 2962, 2930, 2876, 2858, 1733, 1692, 1461, 1259, 1052, 981 $cm^{-1}$; $^{1}H$ NMR ($CDCl_3$, 600 MHz) δ 6.99 (1H, s, H-19), 6.60 (1H, bs, H-17), 5.42 (1H, dd, *J* = 8.0, 3.0 Hz, H-15), 4.25 (1H, ddd, *J* = 9.5, 6.3, 2.8 Hz, H-3), 4.23 (1H, bs, 3-OH), 3.77 (1H, ddd, *J* = 4.0, 3.9, 3.8 Hz, H-7), 3.30 (1H, dq, *J* = 4.0, 6.9 Hz, H-6), 3.01 (2H, q, *J* = 7.6 Hz, H-21), 2.81 (1H, dd, *J* = 7.7, 4.6 Hz, H-13), 2.68 (1H, bs, 7-OH), 2.54 (1H, dd, *J* = 13.9, 9.5 Hz, H-2a), 2.36 (1H, dd, *J* = 13.9, 2.8 Hz, H-2b), 2.11 (1H, ddd, *J* = 15.3, 4.6, 3.0 Hz, H-14a), 2.09 (3H, s, H-27), 1.91 (1H, ddd, *J*= 15.3, 8.0, 7.7 Hz, H-14b), 1.74(1H, m, H-8), 1.73 (1H, m, H-11a), 1.51 (1H, m, H-10a), 1.41 (1H, m, H-11b), 1.39 (3H, t, *J*= 7.6 Hz, H-28), 1.38 (3H, m, H-9 and H-10b), 1.37 (3H, s, H-23), 1.28 (3H, s, H-26), 1.17 (3H, d, *J* = 6.9 Hz, H-24), 1.09 (3H, s, H-22), 1.01 (3H, d, *J* = 7.0 Hz, H-25); EIMS *m/z* 521 $[M]^+$ (22), 449 (7), 350 (18), 334 (57), 248 (16), 234 (27), 196 (41), 182 (59), 180 (96), 178 (100), 166 (44), 154 (44); HREIMS *m/z* 521.2808 (calcd. for $C_{28}H_{43}NO_6S$, 521.2811).

**Epothilone $G_1$ (10):** colorless amorphous solid; $[\alpha]^{22}_D$ -39.7 (*c* 1.0, MeOH); UV (MeOH) $\lambda_{max}$ nm (ε) 203 (15200), 236 (15100); IR (KBr) $\nu_{max}$ 3456, 2962, 2933, 2876, 1736, 1691, 1585, 1466, 1262, 980 $cm^{-1}$; $^{1}H$ NMR ($CDCl_3$, 400 MHz) δ 7.47 (1H, s, H-19), 6.33 (1H, bs, H-17), 5.42 (1H, dd, *J* = 8.3, 2.9 Hz, H-15), 4.11 (1H, ddd, *J* = 10.1, 6.1, 3.4 Hz, H-3), 3.78 (1H, bddd, *J* = 5.2, 3.5, 3.5 Hz, H-7), 3.63 (1H, bd, *J* = 6.1 Hz, 3-OH), 3.21 (1H, dq, *J* = 5.2, 7.0 Hz, H-6), 3.00 (1H, ddd, *J* = 7.7, 4.8, 4.2 Hz, H-13), 2.88 (1H, ddd, *J* = 7.1, 4.2, 4.2 Hz, H-12), 2.53 (1H, dd, *J* = 14.8, 10.1 Hz, H-2a), 2.51 (1H, bd, *J* = 3.5 Hz, 7-OH), 2.43 (1H, dd, *J* = 14.8, 3.4 Hz, H-2b), 2.43 (3H, s, H-21), 2.07 (1H, ddd, *J* = 15.1, 4.8, 2.9 Hz, H-14a), 1.99 (3H, d, *J* = 1.3 Hz, H-27), 1.86 (1H, ddd, *J* = 15.1, 8.3, 7.7 Hz, H-14b), 1.71 (1H, m, H-8), 1.69 (1H, m, H-11a) 1.53 (1H, m, H-10a), 1.42 (1H, m, H-11b), 1.40 (3H, m, H-9 and H-10b), 1.34 (3H, s, H-23), 1.16 (3H, d, *J*= 7.0 Hz, H-24), 1.09 (3H, s, H-22), 0.99 (3H, d, *J*= 6.9 Hz, H-25); $^{13}C$ NMR ($CDCl_3$, 100 MHz) δ 220.1 (s, C-5), 170.5 (s, C-1), 161.0 (s, C-20), 137.4 (s, C-18), 136.7 (s, C-16), 135.9 (d, C-19), 116.4 (d, C-17), 76.4 (d, C-15), 74.9 (d, C-7), 73.7 (d, C-3), 57.4 (d, C-12), 54.4 (d, C-13), 52.6 (s, C-4), 43.8 (d, C-6), 38.8 (t, C-2), 36.2 (d, C-8), 31.4 (t, C-14), 30.4 (t, C-9), 27.0 (t, C-11), 23.9 (t, C-10), 21.3 (q, C-23), 21.2 (q, C-22), 17.2 (q, C-25), 15.8 (q, C-27), 14.4 (q, C-24), 13.8 (q, C-21); EIMS *m/z* 477 $[M]^+$ (4), 405 (7), 290 (40), 152 (39), 150 (100), 148 (23), 124 (23); HREIMS *m/z* 477.2684 (calcd. for $C_{26}H_{39}NO_7$, 477.2727).

**Epothilone $G_2$ (11):** colorless amorphous solid; $[\alpha]^{22}_D$ -22.6 (*c* 1.0, MeOH); UV (MeOH) $\lambda_{max}$ nm (ε) 202 (21500), 236 (14800); IR (KBr) $\nu_{max}$ 3456, 2965, 2934, 2877, 1737, 1690, 1586, 1464, 1250, 980 $cm^{-1}$, $^{1}H$ NMR ($CDCl_3$, 400 MHz) δ 7.48 (1H, s, H-19), 6.33 (1H, bs, H-17), 5.43 (1H, dd, *J* = 7.1, 3.6 Hz, H-15), 4.12 (1H, ddd, *J* = 9.9, 6.4, 3.4 Hz, H-3), 3.77 (1H, ddd, *J* = 4.7, 4.4, 4.1 Hz, H-7), 3.83 (1H, bd, *J* = 6.4 Hz, 3-OH), 3.30 (1H, dq, *J* = 4.7, 6.9 Hz, H-6), 2.78 (1H, dd, *J*= 7.0, 5.4 Hz, H-13), 2.54 (1H, dd, *J* = 14.3, 9.9 Hz, H-2a), 2.51 (1H, bd, *J* = 4.1 Hz, 7-OH), 2.44 (3H, s, H-21), 2.40 (1H, dd, *J* = 14.3, 3.4 Hz, H-2b), 2.03 (1H, ddd, *J* = 15.2, 5.4, 3.6 Hz, H-14a), 2.00 (3H, d, *J*= 1.3 Hz, H-27), 1.92 (1H, ddd, *J* = 15.1, 7.1, 7.0 Hz, H-14b), 1.71 (1H, m, H-8), 1.68 (1H, m, H-11a), 1.51 (1H, m, H-10a), 1.42 (1H, m, H-11b), 1.39 (3H, m, H-9 and H-10b), 1.35 (3H, s, H-23), 1.26 (3H, s, H-26), 1.16 (3H, d, *J*= 6.9 Hz, H-24), 1.07 (3H, s, H-22), 0.99 (3H, d, *J*= 7.0 Hz, H-25); $^{13}C$ NMR ($CDCl_3$, 100 MHz) δ 220.7 (s, C-5), 170.5 (s, C-1), 161.0 (s, C-20), 137.4 (s, C-18), 136.5 (s, C-16), 135.9 (d, C-19), 116.3 (d, C-17), 76.6 (d, C-15), 74.6 (d, C-7), 73.5 (d, C-3), 61.3 (s, C-12), 61.1 (d, C-13), 52.7 (s, C-4), 43.4 (d, C-6), 39.0 (t, C-2), 36.5 (d, C-8), 32.0 (t, C-11), 31.8 (t, C-14), 30.8 (t, C-9), 22.8 (t, C-10), 22.9 (q, C-26), 21.0 (q, C-23), 20.8 (q, C-22), 17.2 (q, C-25), 15.9 (q, C-27), 14.1 (q, C-24), 13.8 (q, C-21); EIMS *m/z* 491$[M]^+$ (21), 419 (6), 320 (18), 304 (39), 166 (42), 152 (57), 150 (100), 149 (44), 148 (58), 124 (35), 109 (33); HREIMS *m/z* 491.2878 (calcd. for $C_{27}H_{41}NO_7$, 491.2883).

**Epothilone $H_1$ (12):** colorless amorphous solid; $[\alpha]^{22}_D$ -84.2 (*c* 0.2, MeOH); UV (MeOH) $\lambda_{max}$ nm (ε) 203 (19600), 237 (12000); IR (KBr) $\nu_{max}$ 3436, 2933, 2880, 2860, 1734, 1688, 1585, 1251, 1007 $cm^{-1}$; $^{1}H$ NMR ($CDCl_3$, 400 MHz) δ 7.47 (1H, s, H-19), 6.31 (1H, bs, H-17), 5.43 (1H, ddd, *J* = 10.6, 10.2, 4.5 Hz, H-12), 5.36 (1H, dddd, *J*= 10.6, 9.6, 5.0, 1.3 Hz, H-13), 5.30 (1H, dd, *J* = 9.9, 2.0 Hz, H-15), 4.16 (1H, ddd, *J* = 11.2, 5.3, 2.8 Hz, H-3), 3.73 (1H, ddd, *J* = 3.9, 2.5, 2.3 Hz, H-7), 3.12 (1H, dq, *J* = 2.3, 6.9 Hz, H-6), 2.92 (1H, d, *J*= 2.5 Hz, 7-OH), 2.91 (1H, d, *J* = 5.3

Hz, 7-OH), 2.66 (1H, ddd, *J*= 15.1, 9.9, 9.6 Hz, H-14a), 2.50 (1H, dd, *J* = 15.4, 11.2 Hz, H-2a), 2.43 (3H, s, H-21), 2.37 (1H, dd, *J* = 15.4, 2.8 Hz, H-2b), 2.23 (1H, m, H-14b), 2.18 (1H, m, H-11a), 2.01 (1H, m, H-11b), 2.08 (3H, d, *J* = 1.3 Hz, H-27), 1.74 (1H, m, H-8), 1.65 (1H, m, H-10a), 1.33 (1H, m, H-9a), 1.31 (3H, s, H-23), 1.19 (1H, m, H-10b), 1.18 (1H, m, H-9b), 1.17 (3H, d, *J* = 6.9Hz, H-24), 1.08 (3H, s, H-22), 0.99 (3H, d, *J* = 7.1 Hz, H-25); $^{13}$C NMR, see Table 1; EIMS *m*/*z* 461 $[M]^+$ (6), 310 (5), 274 (10), 273 (7), 171 (63), 152 (100), 148 (18), 111 (15); HREIMS *m*/*z* 461.2743 (calcd. for $C_{26}H_{39}NO_6$, 461.2777).

**Epothilone $H_2$ (13):** colorless amorphous solid; $[\alpha]^{22}_D$ -44.4 (*c* 0.25, MeOH); UV (MeOH) $\lambda_{max}$ nm (ε) 203 (14500), 236 (12200); IR (KBr) $\nu_{max}$ 3436, 2967, 2935, 2880, 1734, 1690, 1586, 1251, 1007 cm$^{-1}$; $^1$H NMR ($CDCl_3$, 400 MHz) δ 7.46 (1H, s, H-19), 6.30(1H, bs, H-17), 5.23 (1H, dd, *J* = 9.8, 2.1 Hz, H-15), 5.12 (1H, dd, *J* = 10.1, 5.3 Hz, H-13), 4.20 (1H, ddd, *J* = 10.8, 5.7, 2.9 Hz, H-3), 3.71 (1H, ddd, *J* = 3.8, 2.6, 2.6 Hz, H-7), 3.14 (1H, dq, *J* = 2.6, 6.9 Hz, H-6), 2.93 (d, *J* = 5.7 Hz, 3-OH), 2.90 (1H, bd, *J* = 2.6 Hz, 7-OH), 2.62 (1H, ddd, *J* = 15.1, 9.8, 9.8 Hz, H-14a), 2.46 (1H, dd, *J* = 15.1, 10.8 Hz, H-2a), 2.43 (3H, s, H-21), 2.32 (1H, dd, *J* = 15.1, 2.9 Hz, H-2b), 2.29 (1H, m, H-11a), 2.19 (1H, bd, *J* = 15.1 Hz, H-14b), 1.97 (3H, d, *J*= 1.3 Hz, H-27), 1.87 (1H, m, H-11b), 1.73 (1H, m, H-8), 1.67 (1H, m, H-10a), 1.65 (3H, bs, H-26), 1.32 (3H, s, H-23), 1.26 (2H, m, H-9), 1.24 (1H, m, H-10b), 1.18 (3H, d, *J* = 6.9 Hz, H-24), 1.07 (3H, s, H-22), 1.00 (3H, d, *J* = 7.0 Hz, H-25); $^{13}$C NMR ($CDCl_3$, 100 MHz) δ 220.6 (s, C-5), 170.3 (s, C-1), 161.0 (s, C-20), 138.6 (s, C-12), 138.4 (s, C-16), 137.5 (s, C-18), 135.6 (d, C-19), 120.8 (d, C-13), 115.8 (d, C-17), 78.9 (d, C-15), 74.3 (d, C-7), 72.7 (d, C-3), 53.3 (s, C-4), 42.0 (d, C-6), 39.6 (t, C-2), 38.6 (d, C-8), 32.4 (t, C-14), 31.9 (t, C-9), 31.6 (t, C-11), 25.6 (t, C-10), 23.0 (q, C-26), 22.8 (q, C-23), 18.8 (q, C-22), 16.1 (q, C-27), 15.9 (q, C-25), 13.8 (q, 0.21), 13.6 (q, C-24); EIMS *m*/*z* 475 $[M]^+$ (11), 288 (9), 287 (5), 188(7), 171 (32), 152 (100), 111 (10); HREIMS *m*/*z* 475.2913 (calcd. for $C_{27}H_{41}NO_6$, 475.2934).

**Epothilone $C_1$ (16):** colorless amorphous solid; $[\alpha]^{22}_D$ -114.0 (*c* 10.0, MeOH); UV (MeOH) $\lambda_{max}$ nm (ε) 211 (16500), 248 (12500); IR (KBr) $\nu_{max}$ 3440, 2933, 2877, 2858, 1730, 1708, 1457, 1244, 981 cm$^{-1}$; $^1$H NMR ($CDCl_3$, 300 MHz) δ 6.96 (1H, s, H-19), 6.56 (1H, bs, H-17), 5.47 (1H, dd, *J* = 9.2, 3.0 Hz, H-15), 5.43 (1H, m, H-12), 5.40 (1H, m, H-13), 4.40 (1H, ddd, *J* = 6.2, 6.1, 6.1 Hz, H-3), 3.69 (1H, dd, *J*= 5.7, 3.6 Hz, H-7), 3.01 (1H, dq, *J*= 5.7, 6.9 Hz, H-6), 3.01 (1H, bs, 3-OH), 2.84 (1H, dq, *J*= 5.2, 7.0 Hz, H-4), 2.68 (3H, s, H-21), 2.66 (1H, ddd, *J* = 16.4, 9.2, 7.3 Hz, H-14a), 2.64 (1H, dd, *J* = 15.9, 7.1 Hz, H-2a), 2.54 (1H, dd, *J*= 15.9, 6.1 Hz, H-2b), 2.38 (1H, bd, *J*= 16.4 Hz, H-14b), 2.35 (1H, bs, 7-OH), 2.07 (3H, bs, H-27), 2.03 (2H, m, H-11), 1.62 (1H, m, H-10a), 1.53 (1H, m, H-8), 1.35 (1H, m, H-9a), 1.22 (1H, m, H-9b), 1.19(3H, d, *J* = 6.9 Hz, H-24), 1.14(3H, d, *J* = 6.9 Hz, H-23), 1.10(1H, m, H-10b), 0.95 (3H, d, *J* = 6.9 Hz, H-25); $^{13}$C NMR, see Table 1; EIMS *m*/*z* 463 $[M]^+$ (5), 324 (8), 290 (8), 204 (7), 168(100), 164 (15), 139 (36); HREIMS *m*/*z* 463.2381 (calcd. for $C_{25}H_{37}NO_5S$, 463.2392).

**Epothilone $D_1$ (17):** colorless amorphous solid; $[\alpha]^{22}_D$ -118.6 (*c* 0.5, MeOH); UV (MeOH) $\lambda_{max}$ nm (ε) 208 (18300), 249 (11900); IR (KBr) $\nu_{max}$ 3439, 2965, 2934, 2877, 1729, 1707, 1456, 1250, 980 cm$^{-1}$; $^1$H NMR ($CDCl_3$, 300 MHz) δ 6.98 (1H, s, H-19), 6.56 (1H, bs, H-17), 5.51 (1H, dd, *J*= 9.5, 3.4 Hz, H-15), 5.16 (1H, dd, *J*= 8.0, 4.2 Hz, H-13), 4.42 (1H, ddd, *J*= 7.1, 6.3, 5.5 Hz, H-3), 3.70 (1H, dd, *J*= 6.5, 2.9 Hz, H-7), 3.07 (1H, dq, *J*= 6.5, 6.9 Hz, H-6), 2.95 (1H, dq, *J*= 4.7, 7.0 Hz, H-4), 2.71 (3H, s, H-21), 2.69 (1H, dd, *J* = 16.0, 6.3 Hz, H-2a), 2.64 (1H, m, H-14a), 2.59 (1H, dd, *J*= 16.0, 7.1 Hz, H-2b), 2.46 (1H, bs, 3-OH), 2.38 (1H, bd, *J* = 16.0 Hz, H-14b), 2.19 (1H, ddd, *J*= 13.3, 8.6, 5.7 Hz, H-11a), 2.10 (3H, d, *J* = 1.4 Hz, H-27), 2.02 (1H, bs, 7-OH), 1.91 (1H, ddd, *J* = 13.3, 6.0, 6.0 Hz, H-11b), 1.68 (1H, m, H-10a), 1.66 (3H, bs, H-26), 1.53 (1H, m, H-8), 1.37 (1H, m, H-9a), 1.26 (1H, m, H-9b), 1.24 (3H, d, *J* = 6.9 Hz, 11-24), 1.19 (1H, m, H-10b), 1.14 (3H, d. *J* = 7.0, H-23), 0.99 (3H, d, *J* = 6.9 Hz, H-25); $^{13}$CNMR ($CDCl_3$, 100 MHz) δ 217.0 (s, C-5), 169.7 (s, C-1), 165.0 (s, C-20), 152.2 (s, C-18), 138.5 (s, C-12), 137.7 (s, C-16), 120.7 (d, C-13), 120.1 (d, C-17), 116.3 (d, C-19), 78.8 (d, C-15), 77.2 (d, C-7), 67.7 (d, C-3), 52.1 (d, C-4), 46.5 (d, C-6), 40.6 (t, C-2), 37.6 (d, C-8), 32.3 (t, C-14), 31.8 (t, C-11), 29.5(t, C-9), 25.5 (t, C-10), 23.1 (q, C-26), 19.2 (q, C-21), 15.5 (q, C-27), 16.6 (q, C-25), 14.5 (q, C-24), 9.7 (q, C-23); EIMS *m*/*z* 477 $[M]^+$ (13), 304 (19), 303 (31), 218 (40), 204 (41), 168 (100), 164 (45), 157 (25), 139 (18); HREIMS *m*/*z* 477.2544 (calcd. for $C_{26}H_{39}NO_5S$, 477.2549).

**Epothilone $C_2$ (18):** colorless amorphous solid; $[\alpha]^{22}_D$ -11.6 (*c* 10.0, MeOH); UV (MeOH) $\lambda_{max}$ nm (ε) 212 (15500), 249 (12100); IR (KBr) $\nu_{max}$ 3428, 2962, 2929, 2877, 2859, 1734, 1705, 1460, 1251, 982 cm$^{-1}$; $^1$H NMR ($CDCl_3$, 300 MHz) δ 6.99 (1H, s, H-19), 6.66 (1H, bs, H-17), 5.55 (1H, ddd, *J* = 10.4, 9.2, 6.1 Hz, H-12), 5.38 (1H, ddd, *J* = 10.4, 9.3, 6.2 Hz, H-13), 5.22 (1H, dd, *J* = 8.8, 2.8 Hz, H-15), 4.42 (1H, dddd, *J*= 9.4, 5.6, 4.2, 4.1 Hz, H-3), 3.93 (1H, d, *J*= 5.6 Hz, 3-OH), 3.86 (1H, m, H-7), 3.15 (1H, bs, 7-OH), 3.12 (1H, dq, *J* = 4.2, 7.0 Hz, H-4), 3.00 (1H, dq, *J* = 6.9, 7.0 Hz, H-6), 2.70 (3H, s, H-21), 2.62 (1H, dddd, *J*= 15.1, 9.3, 8.8, 0.8 Hz, H-14a), 2.58 (1H, dd, *J*= 15.4, 9.4 Hz, H-2a), 2.38 (1H, dd, *J*= 15.4, 4.1 Hz, H-2b), 2.31 (1H, ddd, *J* = 15.1, 6.2, 2.8 Hz, H-14b), 2.08 (3H, d, *J*= 1.3 Hz, H-27), 2.15 (1H, m, H-11a), 2.04 (1H, m, H-11b), 1.71 (1H, m, H-8), 1.59 (1H, m, H-10a), 1.43 (1H, m, H-9a), 1.31 (1H, m, H-9b), 1.26 (3H, d, *J* = 7.0 Hz, H-24), 1.15 (3H, d, *J*= 7.0 Hz, H-23), 1.11 (1H, m, H-10b), 1.00 (3H, d, *J* = 6.9 Hz, H-25); $^{13}$C NMR, see Table 1; EIMS *m*/*z* 463 $[M]^+$ (7), 324 (7), 306 (8), 290 (17), 168 (100), 164 (14), 139 (27); HREIMS *m*/*z* 463.2392 (calcd. for $C_{25}H_{37}NO_5S$, 463.2392).

**Epothilone $D_2$ (19):** colorless amorphous solid; $[\alpha]^{22}_D$-12.5 (*c* 1.0, MeOH); UV (MeOH) $\lambda_{max}$ nm (ε) 210 (15400), 248 (11200); IR (KBr) $\nu_{max}$ 3436, 2965, 2930, 2877, 1732, 1705, 1458, 1253, 980 cm$^{-1}$; $^1$H NMR ($CDCl_3$, 400 MHz) δ 6.97 (1H, s, H-19), 6.56 (1H, bs, H-17), 5.18 (1H, dd, *J* = 7.9, 4.9 Hz, H-15), 5.18 (1H, ddd, *J*= 9.6, 5.4, 1.0 Hz,

H-13), 4.27 (1H, m, H-3), 3.88 (1H, dd, *J* = 5.6, 4.6 Hz, H-7), 3.19 (1H, bs, 3-OH), 3.07 (1H, dq, *J*= 4.3, 7.0 Hz, H-4), 2.95 (1H, dq, *J* = 5.6, 7.0 Hz, H-6), 2.70 (3H, s, H-21), 2.62 (1H, dd, *J*= 14.9, 7.8 Hz, H-2a), 2.56 (1H, ddd, *J* = 14.7, 9.6, 7.9 Hz, H-14a), 2.43 (1H, dd, *J*= 14.9, 5.6 Hz, H-2b), 2.38 (1H, bs, 7-OH), 2,26 (1H, ddd, *J* = 14.5, 5.4, 4.9 Hz, H-14b), 2.19 (1H, ddd, *J*= 13.0, 10.4, 5.4 Hz, H-11a), 2.10 (3H, d, *J* = 1.4 Hz, H-27), 1.95 (1H, ddd, *J* = 13.0, 10.3, 5.3 Hz, H-11b), 1.72 (1H, m, H-8), 1.68 (3H, bs, H-26), 1.61 (1H, m, H-10a), 1.39 (2H, m, H-9), 1.21 (1H, m, H-10b), 1.19 (3H, d, *J* = 6.9 Hz, H-24), 1.17 (3H, d. *J* = 7.0, H-22), 1.00 (3H, d, *J* = 6.9 Hz, H-25); $^{13}C$ NMR ($CDCl_3$, 100 MHz) δ 216.8 (s, C-5), 170.4 (s, C-1), 164.9 (s, C-20), 152.3 (s, C-18), 139.8 (s, C-12), 137.5 (s, C-16), 120.5 (d, C-17), 119.2 (d, C-13), 116.3 (d, C-19), 80.0 (d, C-15), 74.3 (d, C-7), 69.7 (d, C-3), 48.6 (d, C-4), 48.4 (d, C-6), 39.9 (t, C-2), 36.6 (d, C-8), 32.2 (t, C-14), 32.7 (t, C-11), 30.9 (t, C-9), 26.0 (t, C-10), 23.6 (q, C-26), 19.2 (q, C-21), 15.4 (q, C-27), 17.1 (q, C-25), 12.4 (q, C-24),12.7 (q, C-23); EIMS *m/z* 477 $[M]^+$ (22), 304 (19), 303 (17), 218 (22), 204 (25), 168 (100), 164 (28), 157 (31), 139 (21); HREIMS *m/z* 477.2545 (calcd. for $C_{26}H_{39}NO_5S$, 477.2549).

**Epothilone $C_3$ (20):** colorless amorphous solid; $[\alpha]^{22}_D$-62.1 (*c* 5.0, MeOH); UV (MeOH) $\lambda_{max}$ nm (ε) 212 (16200), 248 (12300); IR (KBr) $\nu_{max}$ 3432, 2928, 2878, 2858, 1736, 1698, 1252, 1040 $cm^{-1}$; $^1H$ NMR ($CDCl_3$, 300 MHz) δ 6.95 (1H, s, H-19), 6.56 (1H, bs, H-17), 5.44 (1H, ddd, *J* = 10.9, 10.3, 5.4 Hz, H-12), 5.33 (1H, ddd, *J*= 10.9, 9.3, 4.6 Hz, H-13), 5.23 (1H, dd, *J* = 9.5, 2.2 Hz, H-15), 4.36 (1H, ddd, *J*= 11.3, 5.6, 2.3 Hz, H-3), 4.04 (1H, d, *J*= 5.6 Hz, 3-OH), 3.93 (1H, ddd, *J* = 9.5, 2.3, 1.4 Hz, H-7), 3.56 (1H, bd, *J*= 2.3 Hz, 7-OH), 2.70 (1H, dd, *J* = 18.0, 1.4 Hz, H-6a), 2.67 (3H, s, H-21), 2.61 (1H, ddd, *J* = 15.3, 9.5, 9.3 Hz, H-14a), 2.38 (1H, dd, *J* = 14.3, 11.3 Hz, H-2a), 2.36 (1H, dd, *J* = 18.0, 9.5 Hz, H-6b), 2.28 (1H, bd, *J* = 15.3 Hz, H-14b), 2.12 (1H, m, H-11a), 2.06 (1H, dd, *J* = 14.3, 2.3 Hz, H-2b), 2.03 (3H, d, *J* = 1.3 Hz, H-27), 1.96 (1H, m, H-11b), 1.75 (1H, m, H-8), 1.54 (1H, m, H-10a), 1.26 (1H, m, H-9a), 1.25 (3H, s, H-23), 1.17 (1H, m, H-10b), 1.15 (1H, m, H-9b), 1.03 (3H, s, H-22), 0.91 (3H, d, *J*= 6.8 Hz, H-25); $^{13}C$ NMR, see Table 1; EIMS *m/z* 463 $[M]^+$ (28), 290 (14), 168 (100), 164 (36), 157 (44), 151 (25); HREIMS *m/z* 463.2379 (calcd. for $C_{25}H_{37}NO_5S$, 463.2392).

**Epothilone $C_4$ (21)**: colorless amorphous solid; $[\alpha]^{22}_D$-75.6 (*c* 1.0, MeOH); UV (MeOH) $\lambda_{max}$ nm (ε) 212 (17200), 248 (12500); IR(KBr) $\nu_{max}$ 3434, 2974, 2932, 2859, 1735, 1686, 1252, 1046 $cm^{-1}$; $^1H$ NMR ($CDCl_3$, 300 MHz) δ 6.96 (1H, s, H-19), 6.60 (1H, bs, H-17), 5.43 (1H, m, H-12), 5.40 (1H, m, H-13), 5.26 (1H, dd, *J*= 9.6, 2.3 Hz, H-15), 4.41 (1H, ddd, *J* = 11.4, 5.8, 2.5 Hz, H-3), 3.78 (1H, m, H-7), 3.70 (1H, bs, 3-OH), 3.46 (1H, d, *J*= 0.9 Hz, 7-OH), 3.01 (1H, dq, *J*= 0.5, 7.0 Hz, H-6), 2.69(3H, s, H-21), 2.66 (1H, ddd, *J*= 15.3, 9.6, 8.8 Hz, H-14a), 2.47 (1H, dd, *J* = 14.5, 11.4 Hz, H-2a), 2.29 (1H, m, H-14b), 2.25 (1H, dd, *J*= 14.5, 2.5 Hz, H-2b), 2.24 (1H, m, H-11a), 2.07 (3H, d, *J*= 1.4 Hz, H-27), 1.96 (1H, m, H-11b), 1.51 (2H, m, H-8), 1.44 (2H, m, H-10), 1.37 (2H, m, H-9), 1.32 (3H, s, H-23), 1.17 (3H, d, *J* = 7.0 Hz, H-24), 1.07 (3H, s, H-22); $^{13}C$ NMR, see Table 1; EIMS *m/z* 463 $[M]^+$ (7), 276 (15), 171 (33), 168 (100), 164 (23), 151 (22), 111 (13); HREIMS *m/z* 463.2373 (calcd. for $C_{25}H_{37}NO_5S$, 463.2392).

**Epothilone $C_5$ (22):** colorless amorphous solid; $[\alpha]^{22}_D$ -158.2 (*c* 0.5, MeOH); UV (MeOH) $\lambda_{max}$ nm (ε) 205 (19500), 247 (12700); IR (KBr) $\nu_{max}$ 3447, 2972, 2927, 1737, 1690, 1450, 1252, 1181, 986 $cm^{-1}$; $^1H$ NMR ($CDCl_3$, 400 MHz) δ 6.93 (1H, s, H-19), 6.48 (1H, bs, H-17), 5.48 (1H, ddd, *J* = 10.7, 6.2, 6.2 Hz, H-12), 5.39 (1H, m, H-13), 5.37 (1H, m, H-9), 5.34 (1H, dd, *J* = 8.0, 2.3 Hz, H-15), 4.29 (1H, dd, J= 6.0, 2.6 Hz, H-7), 4.09 (1H, ddd, *J* = 10.8, 7.1, 2.9 Hz, H-3), 3.59 (1H, d, *J* = 7.1 Hz, 3-OH), 3.17 (1H, dq, *J*= 6.0, 6.9 Hz, H-6), 2.68 (3H, s, H-21), 2.54 (1H, ddd, *J* = 15.2, 8.1, 8.0 Hz, H-14a), 2.44 (1H, bs, 7-OH), 2.42 (1H, dd, *J* = 15.1, 2.9 Hz, H-2a), 2.41 (1H, ddd, *J* = 15.2, 2.3, 2.3 Hz, H-14b), 2.34 (1H, dd, *J* = 15.1, 10.8 Hz, H-2b), 2.20 (1H, m, H-10a), 2.18 (2H, m, H-11), 2.12 (1H, m, H-10b), 2.06 (3H, bs, H-27), 1.67 (3H, bs, H-25), 1.27 (3H, s, H-23), 1.21 (3H, d, *J* = 6.9 Hz, H-24), 1.15 (3H, s, H-22); $^{13}C$ NMR, see Table 1; EIMS *m/z* 475 $[M]^+$ (6), 392 (7), 304 (6), 288 (33), 204 (76), 171 (19), 168 (100), 164 (12); HREIMS *m/z* 475.2380 (calcd. for $C_{26}H_{37}NO_5S$, 475.2392).

**Epothilone $D_5$ (23)**: colorless amorphous solid; $[\alpha]^{22}_D$-150 (*c* 0.2, MeOH); UV (MeOH) $\lambda_{max}$ nm (ε) 205 (23300), 248 (13600); IR(KBr) $\nu_{max}$ 3439, 2967, 2927, 1736, 1690, 1451, 1254, 1181, 987 $cm^{-1}$; $^1H$ NMR ($CDCl_3$, 400 MHz) δ; 6.94 (1H, s, H-19), 6.51 (1H, bs, H-17), 5.34 (1H, bs, H-9), 5.29 (1H, dd, *J* = 8.0, 2.4 Hz, H-15), 5.16 (1H, dd, *J* = 8.2, 6.2 Hz, H-13), 4.30 (1H, bd, *J* = 4.9 Hz, H-7), 4.19 (1H, ddd, *J* = 10.8, 7.6, 3.0 Hz, H-3), 3.68 (1H, d, *J* = 7.6 Hz, 3-OH), 3.17 (1H, dq, *J* = 4.9, 7.0 Hz, H-6), 2.69 (3H, s, H-21), 2.65 (1H, d, *J* = 2.1 Hz, 7-OH), 2.56 (1H, ddd, *J* = 16.2, 8.2, 8.0 Hz, H-14a), 2.40 (1H, dd, *J* = 15.0, 3.0 Hz, H-2a), 2.39 (1H, bd, *J* = 16.2 Hz, H-14b), 2.34 (1H, dd, *J* = 15.0, 10.8 Hz, H-2b), 2.25 (2H, m, H-10a and H-11a), 2.20 (1H, m, H-10b), 2.17 (1H, m, H-11b), 2.05 (3H, d, *J* = 1.0 Hz, H-27), 1.69 (3H, bs, H-25), 1.68 (3H, bs, H-26), 1.29 (3H, s, H-23), 1.23 (3H, d, *J* = 7.0 Hz, H-24), 1.16 (3H, s, H-22); $^{13}C$ NMR, see Table 1; EIMS *m/z* 489 $[M]^+$ (4), 406 (4), 338 (7), 302 (13), 218 (35), 171 (10), 168 (100), 153 (20), 125 (10); HREIMS *m/z* 489.2536 (calcd. for $C_{27}H_{39}NO_5S$, 489.2549).

**Epothilone $C_6$ (24)**: colorless amorphous solid; $[\alpha]^{22}_D$ -205.2 (*c* 1.0, MeOH); UV (MeOH) $\lambda_{max}$ nm (ε) 218 (24600), 237 (28800); IR (KBr) $\nu_{max}$ 3435, 2967, 2927, 2882, 1732, 1688, 1465, 1258, 988 $cm^{-1}$; $^1H$ NMR ($CDCl_3$, 300 MHz) δ 6.97 (1H, s, H-19), 6.58 (1H, bs, H-17), 6.43 (1H, dd, 15.5, 10.8 Hz, H-11), 6.11 (1H, dd, *J*= 10.8, 10.6 Hz, H-12), 5.75 (1H, ddd, *J*= 15.5, 8.3, 5.6 Hz, H-10), 5.34 (1H, m, H-13), 5.34 (1H, dd, *J* = 9.7, 2.4 Hz, H-15), 4.16 (1H, ddd, *J* = 9.2, 4.9, 4.3 Hz, H-3), 3.74 (1H, ddd, *J* = 2.2, 2.1, 1.7 Hz, H-7), 3.24 (1H, dq, *J* = 2.1, 6.9 Hz, H-6), 3.06 (1H, d, *J*= 2.2 Hz, 7-OH), 2.93 (1H, d, *J* = 4.9 Hz, 3-OH), 2.78 (1H, dddd, *J* = 14.1, 9.9 9.7, 0.7, H-14a), 2.71 (3H, s, H-21), 2.48 (1H, m, H-9a), 2.47 (1H, dd, *J* = 15.5, 9.2 Hz, H-2a), 2.40 (1H, dd, *J* = 15.5, 4.3 Hz, H-2b), 2.38 (1H, bdd, *J* = 14.1, 7.8

Hz, H-14b), 2.11 (3H, d, *J* = 1.3 Hz, H-27), 1.96 (1H, m, H-8), 1.33 (3H, s, H-23), 1.11 (3H, d, *J* = 6.9 Hz, H-24), 1.06 (3H, s, H-22), 1.05 (3H, d, *J* = 6.8 Hz, H-25); $^{13}C$ NMR, see Table 1; EIMS *m/z* 475 $[M]^+$ (13), 387 (2), 316 (4), 288 (15), 230 (16), 204 (9), 171 (18), 168 (100), 164 (14), 151 (17); HREIMS *m/z* 475.2361 (calcd. for $C_{26}H_{37}NO_5S$, 475.2392).

**Epothilone $C_7$ (25):** colorless amorphous solid; $[\alpha]^{22}_D$ **-XXX (*c* 2.0, MeOH); UV (MeOH) $\lambda_{max}$ nm (ε) XXX (XXX), XXX (XXX); IR (KBr) $\nu_{mar}$ XXX $cm^{-1}$;** $^1H$ NMR ($CDCl_3$, 400 MHz) δ 7.01 (1H, s, H-19), 6.66 (1H, bs, H-17), 5.59 (1H, ddd, *J*= 11.1, 11.1, 3.8 Hz, H-12), 5.40 (1H, dd, *J* = 11.1, 9.2, H-13), 5.03 (1H, d, *J* = 9.3 Hz, H-15), 4.62 (1H, dd, *J* = 9.3, 9.2 Hz, H-14), 4.18 (1H, bd, *J* = 11.0 Hz, H-3), 3.72 (1H, bs, H-7), 3.20 (1H, bs, 3-OH), 3.09 (1H, dq, *J* = 1.9, 6.8 Hz, H-6), 3.00 (1H, bs, 7-OH), 2.69 (3H, s, H-21), 2.47 (1H, dd, *J* = 14.8, 11.0 Hz, H-2a), 2.32 (1H, dd, *J* = 14.8, 2.6 Hz, H-2b), 2.27 (1H, m, H-11a), 2.19 (3H, bs, H-27), 2.13 (1H, m, H-11b), 1.76 (1H, m, H-8), 1.70 (1H, m, H-10a), 1.35 (1H, m, H-9a), 1.32 (3H, s, H-23), 1.23 (1H, m, H-9b), 1.21 (1H, m, H-10b), 1.18 (3H, d, *J* = 6.8 Hz, H-24), 1.08 (3H, s, H-22), 1.00 (3H, d, *J* = 6.9 Hz, H-25); **EIMS *m/z* 493 $[M]^+$ XXX; HREIMS *m/z* 493.XXX (calcd. for $C_{26}H_{39}NO_6S$, 493.2498).**

**Epothilone $C_8$ (26):** colorless amorphous solid; $[\alpha]^{22}_D$-75.2 (*c* 2.5, MeOH); UV (MeOH) $\lambda_{max}$ nm (ε) 210 (16800), 248 (17800); IR (KBr) $\nu_{max}$ 3443, 2932, 2881, 1734, 1689, 1465, 1255, 1183, 976 $cm^{-1}$; $^1H$ NMR ($CDCl_3$, 300 MHz) δ 6.93 (1H, s, H-19), 6.62 (1H, dd, *J* = 15.6, 0.6 Hz, H-17), 6.49 (1H, dd, *J* = 15.6, 6.6 Hz, H-16), 5.52 (1H, dddd, *J* = 9.5, 6.6, 2.8, 0.6 Hz, H-15), 5.42 (1H, m, H-12), 5.41 (1H, m, H-13), 4.13 (1H, ddd, *J* = 11.0, 5.3, 2.8 Hz, H-3), 3.69 (1H, ddd, *J* = 3.7, 2.8, 2.5 Hz, H-7), 3.11 (1H, dq, *J* = 2.5, 6.8 Hz, H-6), 2.95 (1H, d, *J* = 5.3 Hz, 3-OH), 2.90 (1H, d, *J* = 2.8 Hz, 7-OH), 2.69 (3H, s, H-21), 2.67 (1H, ddd, *J* = 14.9, 9.5, 8.4 Hz, H-14a), 2.48 (1H, dd, *J* = 15.6, 11.0 Hz, H-2a), 2.33 (1H, dd, *J* = 15.6, 2.8 Hz, H-2b), 2.30 (1H, bd, *J* = 14.9 Hz, H-14b), 2.14 (1H, m, H-11a), 2.03 (1H, m, H-11b), 1.71 (1H, m, H-8), 1.63 (1H, m, H-10a), 1.31 (1H, m, H-9a), 1.29 (3H, s, H-23), 1.17 (3H, d, *J* = 6.8 Hz, H-24), 1.16 (1H, m, H-10b), 1.14 (1H, m, H-9b), 1.05 (3H, s, H-22), 0.97 (3H, d, *J* = 7.1 Hz, H-25); $^{13}C$ NMR, see Table 1; EIMS *m/z* 463 $[M]^+$ (21), 310 (10), 276 (21), 171 (83), 154 (100), 150 (27), 111 (18); HREIMS *m/z* 463.2382 (calcd. for $C_{25}H_{37}NO_5S$, 463.2392).

**Epothilone $C_9$ (27)**: colorless amorphous solid; $[\alpha]^{22}_D$ -93.4 (*c* 1.0, MeOH); UV (MeOH) $\lambda_{max}$ nm (ε) 209 (15200), 254 (15700); IR(KBr) $\nu_{max}$ 3416, 2966, 2932, 1736, 1689, 1463, 1249, 1011 $cm^{-1}$; $^1H$ NMR ($CDCl_3$, 400 MHz) δ 7.06 (1H, s, H-19), 6.65 (1H, bs, H-17), 6.56 (1H, dd, *J* = 10.6, 4.4 Hz, 27-OH), 5.55 (1H, d, *J* = 6.2 Hz, 3-OH),5.52 (1H, dd, *J* = 11.6, 2.0 Hz, H-15), 5.44 (1H, dddd, *J* = 11.2, 10.7, 3.1, 1.7 Hz, H-12), 5.35 (1H, dddd, *J* = 11.0, 10.7, 3.9, 1.7 Hz, H-13), 4.47 (1H, ddd, *J* = 12.5, 4.4, 1.3 Hz, H-27a), 4.35 (1H, ddd, *J* = 11.7, 6.2, 2.6 Hz, H-3), 4.20 (1H, ddd, *J* = 12.5, 10.6, 0.9 Hz, H-27b), 3.63 (1H, ddd, *J* = 4.6, 1.8, 0.9 Hz, H-7), 3.24 (1H, d, *J* = 1.8 Hz, 7-OH), 3.13 (1H, dq, *J* = 0.9, 6.8 Hz, H-6), 2.80 (1H, ddd, *J* = 14.8, 11.6, 11.0 Hz, H-14a), 2.71 (3H, s, H-21), 2.40 (1H, dd, *J* = 14.4, 11.7 Hz, H-2a), 2.24 (1H, m, H-11a), 2.06 (1H, dd, *J* = 14.4, 2.6 Hz, H-2b), 2.01 (1H, ddd, *J* = 14.8, 3.9, 2.0 Hz, H-14b), 2.00 (1H, m, H-11b), 1.77 (1H, m, H-8), 1.69 (1H, m, H-10a), 1.35 (1H, m, H-9a), 1.35 (3H, s, H-23), 1.19 (1H, m, H-10b), 1.19 (3H, d, *J* = 6.8 Hz, H-24), 1.18 (1H, m, H-9b), 1.01 (3H, d, *J* = 7.1 Hz, H-25), 0.98 (3H, s, H-22); $^{13}C$ NMR, see Table 1; EIMS *m/z* 493 $[M]^+$ (17), 306 (64), 184 (50), 171 (30), 167 (38), 166 (100), 138 (12); HREIMS *m/z* 493.2502 (calcd. for $C_{26}H_{39}NO_6S$, 493.2498).

**trans-Epothilone $C_1$ (28):** colorless amorphous solid; $[\alpha]^{22}_D$ -84 (*c* 0.2, MeOH); UV (MeOH) $\lambda_{max}$ nm (ε) 211 (17400), 248 (12900); IR (KBr) $\nu_{max}$ 3433, 2961, 2933, 2879, 1730, 1708, 1457, 1251, 975 $cm^{-1}$; $^1H$ NMR ($CDCl_3$, 600 MHz) δ 7.00 (1H, s, H-19), 6.64 (1H, bs, H-17), 5.45 (1H, ddd, *J* = 15.2, 6.5, 6.5 Hz, H-12), 5.42 (1H, dd, *J* = 6.4, 3.7 Hz, H-15), 5.35 (1H, dt, *J* = 15.2, 7.1 Hz, H-13), 4.42 (1H, m, H-3), 3.58 (1H, ddd, *J* = 8.1, 7.9, 2.8 Hz, H-7), 3.24 (1H, m, H-6), 3.14 (1H, dq, *J* = 4.0, 6.9 Hz, H-6), 2.92 (1H, d, *J* = 7.9 Hz, 7-OH), 2.71 (3H, s, H-21), 2.71 (2H, m, H-2), 2.53 (2H, m, H-14), 2.17 (1H, d, *J* = 2.17 Hz, 3-OH), 2.11 (1H, m, H-11a), 2.06 (3H, bs, H-27), 1.93 (1H, m, H-11b), 1.68 (1H, m, H-9a), 1.65 (1H, m, H-10a), 1.33 (1H, m, H-8), 1.26 (3H, d, *J* = 6.8 Hz, H-24), 1.16 (1H, m, H-10b), 1.12 (3H, d, *J* = 6.9 Hz, H-22), 1.07 (1H, m, H-9b), 1.00 (3H, d, *J* = 6.8 Hz, H-25); $^{13}C$ NMR, see Table 1; EIMS *m/z* 463 $[M]^+$ (6), 290 (21), 289 (20), 204 (23); 194 (19), 190 (22), 168 (100), 164 (48), 157 (14), 152 (19), 151(17), 139(15), 111 (18); HREIMS *m/z* 463.2371 (calcd. for $C_{25}H_{37}NO_5S$, 463.2392).

**trans-Epothilone $C_2$ (29):** colorless amorphous solid; $[\alpha]^{22}_D$ -3 (*c* 1.5, MeOH); UV (MeOH) $\lambda_{max}$ nm (ε) 211 (15800), 248 (11900); IR (KBr) $\nu_{max}$ 3435, 2963, 2931, 2878, 1731, 1706, 1457, 1273, 979 $cm^{-1}$; $^1H$ NMR ($CDCl_3$, 600 MHz) δ 6.99 (1H, s, H-19), 6.57 (1H, bs, H-17), 5.56 (1H, ddd, *J* = 15.1, 7.4, 7.0 Hz, H-12), 5.41 (1H, ddd, *J* = 15.1, 7.0, 6.9 Hz, H-13), 5.41 (1H, dd, *J* = 7.7, 2.8 Hz, H-15), 4.13 (1H, dddd, *J* = 6.7, 6.2, 5.6, 5.1 Hz, H-3), 3.78 (1H, ddd, *J* = 8.2, 6.5, 1.9 Hz, H-7), 3.18 (1H, d, *J* = 5.6 Hz, 3-OH), 3.06 (1H, dq, *J* = 8.2, 7.1 Hz, H-6), 2.98 (1H, dq, *J* = 6.2, 7.0 Hz, H-4), 2.71 (3H, s, H-21), 2.64 (1H, dd, *J* = 15.1, 6.7 Hz, H-2a), 2.54 (1H, dd, *J* = 15.1, 5.1 Hz, H-2b), 2.44 (2H, m, H-14), 2.22 (1H, dddd, *J* = 13.8, 7.0, 6.2, 2.9 Hz, H-11a), 2.10 (3H, d, *J* = 1.1 Hz, H-27), 2.09 (1H, d, *J* = 6.5 Hz, 7-OH), 1.88 (1H, dddd, *J* = 13.8, 10.9, 7.4, 2.9 Hz, H-11b), 1.65 (1H, m, H-8), 1.63 (1H, m, H-10a), 1.56 (1H, dddd, *J* = 12.7, 12.7, 3.9, 3.9 Hz, H-9a), 1.20 (3H, d, *J* = 7.1 Hz, H-24), 1.15 (3H, d, *J* = 7.0 Hz, H-23), 1.13 (1H, m, H-10b), 1.04 (1H, m, H-9b), 1.01 (3H, d, *J* = 7.0 Hz, H-25); $^{13}C$ NMR, see Table 1; EIMS *m/z* 463 $[M]^+$ (13), 290 (11), 190 (10), 168 (100), 164 (20), 157 (26), 139 (17); HREIMS *m/z* 463.2383 (calcd. for $C_{25}H_{37}NO_5S$, 463.2392).

**Epothilone $I_1$ (30):** colorless amorphous solid; **$[\alpha]^{22}_D$ -XXX (*c* XXX, MeOH); UV (MeOH) $\lambda_{max}$ nm (ε) XXX;**

**IR (KBr) $\nu_{max}$ XXX $cm^{-1}$;** $^{1}H$ NMR ($CDCl_3$, 300 MHz) δ 6.96 (1H, s, H-19), 6.54 (1H, bs, H-17), 5.49 (1H, ddd, *J* = 10.3, 7.3, 7.3 Hz, H-12), 5.33 (1H, dd, *J* = 8.3, 4.4 Hz, H-15), 5.31 (1H, m, H-13), 4.15 (1H, ddd, *J* = 8.0, 5.0, 4.6 Hz, H-3), 3.80 (1H, m, H-7), 3.21 (1H, dq, *J* = 6.0, 6.9 Hz, H-6), 2.89 (1H, d, *J*= 5.0 Hz, 3-OH); 2.70 (3H, s, H-21), 2.65 (1H, ddd, *J* = 15.8, 8.5, 8.3 Hz, H-14a), 2.42 (2H, m, H-2), 2.35 (1H, m, H-14b), 2.27 (1H, bd, *J* = 3.3 Hz, 7-OH), 2.13 (1H, m, H-11a), 2.09 (3H, d, *J* = 1.2 Hz, H-27), 2.00 (1H, m, H-11b), 1.72 (1H, m, H-8), 1.40 (2H, m, H-$10_\beta$), 1.37 (1H, m, H-$9_\beta$a), 1.36 (2H, m, H-$9_\alpha$), 1.32 (3H, s, H-23), 1.27 (3H, m, H-$9_\beta$b and H-$10_\alpha$), 1.13 (3H, d, *J*= 6.9 Hz, H-24), 1.09 (3H, s, H-22), 0.94 (3H, d, *J* = 6.9 Hz, H-25); $^{13}C$ NMR ($CDCl_3$, 75 MHz) δ 221.3 (s, C-5), 171.1 (s, C-1), 164.8 (s, C-20), 152.4 (s, C-18), 137.4 (s, C-16), 133.8 (d, C-12), 124.6 (d, C-13), 120.0 (d, C-17), 116.2 (d, C-19), 78.8 (d, C-15), 74.9 (d, C-7), 74.7 (d, C-3), 51.6 (s, C-4), 43.7 (d, C-6), 38.9 (t, C-2), 34.3 (d, C-8), 31.6 (t, C-14), 29.3 (t, C-$9_\alpha$), 28.6 (t, C-$10_\beta$), 28.2 (t, C-$10_\alpha$), 26.6 (t, C-11), 24.8 (t, C-$9_\beta$), 23.6 (q, C-22), 19.3 (q, C23), 19.3 (q, C-21), 16.5 (q, C-25), 15.5 (q, C-27), 13.7 (q, C-24); **EIMS *m/z* 505 $[M]^+$ XXX; HREIMS *m/z* 505.XXX (calcd. for $C_{28}H_{43}NO_5S$, 505.XXX).**

**Epothilone $I_2$ (31):** colorless amorphous solid; $[\alpha]^{22}_{D}$ **-XXX (*c* XXX, MeOH); UV (MeOH) $\lambda_{max}$ nm (ε) XXX; IR (KBr) $\nu_{max}$ XXX $cm^{-1}$;** $^{1}H$NMR ($CDCl_3$, 300 MHz) δ 6.95 (1H, s, H-19), 6.53 (1H, bs, H-17), 5.40 (1H, m, H-12), 5.38 (1H, dd, *J* = 9.8, 3.3 Hz, H-15), 5.37 (1H, m, H-13), 4.21 (1H, ddd, *J* = 8.6, 3.8, 3.6 Hz, H-3), 3.85 (1H, ddd, *J* = 8.5, 5.8, 2.2 Hz, H-7), 3.18 (1H, dq, *J* = 8.5, 7.0 Hz, H-6), 2.70 (3H, s, H-21), 2.65 (1H, ddd, *J* = 15.2, 9.8, 9.0 Hz, H-14a), 2.51 (1H, d, *J* = 3.6 Hz, 3-OH), 2.37 (2H, m, H-2), 2.32 (1H, bd, *J*= 15.2 Hz, H-14b), 2.09 (3H, d, *J*= 1.3 Hz, H-27), 2.07 (2H, m, H-11), 1.78 (1H, m, H-8), 1.65 (1H, d, *J*= 5.8 Hz, 7-OH), 1.57 (1H, m, H-$10_\beta$a), 1.44 (1H, m, H-$10_\alpha$a), 1.42 (1H, m, H-$9_\beta$), 1.32 (3H, s, H-23), 1.21 (1H, m, H-$10_\beta$b), 1.17 (3H, d, *J* = 7.0 Hz, H-24), 1.13 (2H, m, H-$9_\alpha$), 1.06 (3H, s, H-22), 0.95 (3H, d, *J* = 7.0 Hz,H-$25_\alpha$), 0.91 (3H, d, *J* = 6.5 Hz, H-$25_\beta$), 0.68 (1H, m, H-$10_\alpha$b); $^{13}C$ NMR ($CDCl_3$, 100 MHz) δ 220.4 (s, C-5), 171.3 (s, C-1), **XXX (s, C-20)**, 152.4 (s, C-18), 137.6 (s, C-16), 134.5 (d, C-12), 125.3 (d, C-13), 119.6 (d, C-17), 116.2 (d, C-19), 78.6 (d, C-15), 77.2 (d, C-7), 75.0 (d, C-3), 51.0 (s, C-4), 44.6 (d, C-6), 38.2 (t, C-2), 36.9 (t, C-$9_\alpha$), 34.5 (t, C-$10_\alpha$), 32.6 (d, C-8), 32.0 (t, C-14), 30.0 (d, C-$9_\beta$), 27.4 (t, C-11), 26.6 (t, C-$10_\beta$), 25.0 (q, C-22), 21.5 (q, C-$25_\beta$), 19.3 (q, C-21), 17.9 (q, C-$25_\alpha$), 17.7 (q, C-23), 15.8 (q, C-24), 15.6 (q, C-27); **EIMS *m/z* 519 $[M]^+$ XXX; HREIMS *m/z* 519.XXX (calcd. for $C_{29}H_{45}NO_5S$, 519.XXX).**

**Epothilone $I_3$ (32)**: colorless amorphous solid; $[\alpha]^{22}_{D}$ **-XXX (*c* XXX, MeOH); UV (MeOH) $\lambda_{max}$ nm (ε) XXX; IR (KBr) $\nu_{max}$ XXX $cm^{-1}$;** $^{1}H$ NMR ($CDCl_3$, 400 MHz) δ 6.95 (1H, s, H-19), 6.52 (1H, bs, H-17), 5.32 (1H, dd, *J* = 9.1, 3.0 Hz, H-15), 5.08 (1H, dd, *J* = 8.5, 3.9 Hz, H-13), 4.13 (1H, ddd, *J*= 9.4, 4.3, 3.2 Hz, H-3), 3.81 (1H, m, H-7), 3.18 (1H, dq, *J* = 6.8, 7.0 Hz, H-6), 2.83 (1H, d, *J* = 4.3 Hz, 3-OH), 2.70 (3H, s, H-21), 2.61 (1H, ddd, *J* = 15.8, 9.1, 8.5 Hz, H-14a), 2.43 (1H, dd, *J* = 14.0, 3.2 Hz, H-2a), 2.38 (2H, dd, *J* = 14.0, 9.4 Hz, H-2b), 2.30 (1H, bd, *J* = 15.8 Hz, H-14b), 2.16 (1H, ddd, *J* = 14.1, 8.3, 7.4 Hz, H-11a), 2.08 (3H, d, *J* = 1.0 Hz, H-27), 1.99 (1H, d, *J* = 4.7 Hz, 7-OH), 1.92 (1H, ddd, *J* = 14.1, 6.3, 6.3 Hz, H-11b), 1.82 (1H, m, H-8), 1.67 (3H, s, H-26), 1.51 (1H, m, H-$10_\beta$a), 1.40 (1H, m, H-$9_\beta$), 1.33 (1H, m, H-$10_\beta$b), 1.31 (3H, s, H-23), 1.27 (1H, m, H-$10_\alpha$a), 1.23 (1H, m, H-$9_\alpha$a), 1.16 (3H, d, *J* = 7.0 Hz, H-24), 1.10 (1H, m, H-$9_\alpha$b), 1.07 (3H, s, H-22), 0.95 (3H, d, *J* = 7.0 Hz,H-$25_\alpha$), 0.92 (3H, d, *J* = 6.5 Hz, H-$25_\beta$), 0.75 (1H, m, H-$10_\alpha$b); **EIMS *m/z* 533 $[M]^+$ XXX; HREIMS *m/z* 533.XXX (calcd. for $C_{30}H_{47}NO_5S$, 533.XXX).**

**Epothilone $I_4$ (33):** colorless amorphous solid; **$[\alpha]^{22}_{D}$ -XXX (*c* XXX, MeOH); UV (MeOH) $\lambda_{max}$ nm (ε) XXX; IR (KBr) $\nu_{max}$ XXX $cm^{-1}$;** $^{1}H$ NMR ($CDCl_3$, 400 MHz) δ 6.95 (1H, s, H-19), 6.53 (1H, bs, H-17), 5.47 (1H, dt, *J* = 11.1, 5.8 Hz, H-12), 5.33 (1H, ddd, *J* = 9.2, 3.9, 0.5 Hz, H-15), 5.33 (1H, m, H-13), 4.09 (1H, dddd, *J* = 9.6, 8.1, 4.5, 3.3 Hz, H-3), 3.83 (1H, m, H-7), 3.57 (1H, bs, 3-OH), 2.89 (1H, dq, *J* = 7.4, 7.1 Hz, H-6), 2.83 (1H, dq, *J* = 8.1, 7.1 Hz, H-4), 2.70(3H, s, H-21), 2.64 (1H, m, H-14a), 2.42 (1H, dd, *J* = 14.2, 3.3 Hz, H-2a), 2.43 (1H, dd, *J* = 14.2, 9.6 Hz, H-2b), 2.30 (1H, m, H-14b), 2.10 (3H, d, *J* = 1.3 Hz, H-27), 2.09 (2H, m, H-11), 1.81 (1H, m, H-8), 1.74 (1H, bd, *J*=5.6 Hz, 7-OH), 1.53 (1H, m, H-$10_\beta$a), 1.49 (1H, m, H-$9_\beta$), 1.47 (1H, m, H-$10_\alpha$a), 1.27 (1H, m, H-$10_\beta$b), 1.24 (1H, m, H-$9_\alpha$a), 1.17 (3H, d, *J*=7.1 Hz, H-23), 1.14 (1H, m, H-$9_\alpha$b), 1.08 (3H, d, *J*= 7.1 Hz, H-24), 0.97 (3H, d, *J*=6.9 Hz,H-$25_\alpha$), 0.91 (3H, d; *J* = 6.5 Hz, H-$25_\beta$), 0.79 (1H, m, H-$10_\alpha$b); $^{13}C$ NMR ($CDCl_3$, 100 MHz) δ 217.0 (s, C-5), 170.8 (s, C-1), 164.8 (s, C-20), 152.4 (s, C-18), 137.1 (s, C-16), 134.6 (d, C-12), 124.7 (d, C-13), 120.2 (d, C-17), 116.4 (d, C-19), 78.7 (d, C-15), 76.4 (d, C-7), 71.3 (d, C-3), 50.7 (d, C-4), 50.1 (d, C-6), 40.7 (t, C-2), 38.5 (t, C-$9_\alpha$), 35.5 (t, C-$10_\alpha$), 33.4 (d, C-8), 31.8 (t, C-14), 30.0 (d, C-$9_\beta$), 27.2 (t, C-11), 26.7 (t, C-$10_\beta$), 21.4 (q, C-$25_\beta$), 19.3 (q, C-21), 18.2 (q, C-$25_\alpha$), 15.4 (q, C-27), 14.4 (q, C-24), 13.1 (q, C-23); **EIMS *m/z* 505 $[M]^+$ XXX; HREIMS *m/z* 505.XXX (calcd. for $C_{28}H_{43}NO_5S$, 505.XXX).**

**Epothilone $I_5$ (34):** colorless amorphous solid; $[\alpha]^{22}_{D}$**-XXX (*c* XXX, MeOH); UV (MeOH) $\lambda_{max}$ nm (ε) XXX; IR (KBr) $\nu_{max}$ XXX $cm^{-1}$;** $^{1}H$ NMR ($CDCl_3$, 400 MHz) δ 6.97 (1H, s, H-19), 6.52 (1H, bs, H-17), 5.32 (1H, dd, *J* = 7.1, 6.2 Hz, H-15), 5.03 (1H, dd, *J* = 8.4, 5.0 Hz, H-13), 4.05 (1H, dddd, *J* = 7.5, 7.2, 5.9, 4.6 Hz, H-3), 3.91 (1H, m, H-7), 3.17 (1H, d, *J* = 5.9 Hz, 3-OH), 2.94 (1H, dq, *J* = 7.2, 7.1 Hz, H-4), 2.87 (1H, dq, *J* = 6.5, 6.9 Hz, H-6), 2.70 (3H, s, H-21), 2.62 (1H, dd, *J* = 14.6, 4.6 Hz, H-2a), 2.60 (1H, m, H-14a), 2.53 (1H, dd, *J*= 14.6, 7.5 Hz, H-2b), 2.31 (1H, m, H-14b), 2.10 (3H, d, *J* = 1.1 Hz, H-27), 2.10 (1H, m, H-11a), 2.02 (1H, m, H-11b), 1.97 (1H, bd, *J* = 5.6 Hz, 7-OH), 1.84 (1H, m, H-8), 1.66 (3H, s, H-26), 1.55 (1H, m, H-$9_\beta$), 1.49 (1H, m, H-$10_\beta$a), 1.39 (1H, m, H-$10_\beta$b), 1.33(1H, m, H-$10_\alpha$a), 1.31 (1H, m, H-$9_\alpha$a), 1.15 (3H, d, *J* = 7.1 Hz, H-23), 1.12 (1H, m, H-$9_\alpha$b), 1.11 (3H, d, *J* = 6.9 Hz, H-24), 0.97 (3H, d, *J* = 6.9 Hz,H-$25_\alpha$), 0.94 (1H, m, H-$10_\alpha$b), 0.93 (3H, d, *J*= 6.6 Hz, H-$25_\beta$); **EIMS *m/z* 519 $[M]^+$ XXX; HREIMS *m/z* 519.XXX (calcd. for $C_{29}H_{45}NO_5S$, 519.XXX).**

**Epothilone $I_6$ (35):** colorless amorphous solid; **$[\alpha]^{22}_D$ -XXX (*c* XXX, MeOH); UV (MeOH) $\lambda_{max}$ nm ($\epsilon$) XXX; IR (KBr) $\nu_{max}$ XXX $cm^{-1}$;** $^1H$ NMR ($CDCl_3$, 400 MHz) $\delta$ 6.97 (1H, s, H-19), 6.52 (1H, bs, H-17), 5.24 (1H, dd, *J* = 6.9, 6.9 Hz, H-15), 5.02 (1H, dd, *J* = 8.8, 5.2 Hz, H-13), 4.22 (1H, tdd, *J* = 6.1, 5.6, 4.8 Hz, H-3), 3.76 (1H, ddd, *J*= 6.1, 5.7, 5.6 Hz, H-7), 3.13 (1H, d, *J*= 5.6 Hz, 3-OH), 3.05 (1H, dq, *J*= 4.8, 7.0 Hz, H-4), 2.79 (1H, dq, *J*= 5.6, 6.9 Hz, H-6), 2.70 (3H, s, H-21), 2.62 (1H, m, H-14a), 2.57 (2H, d, *J*= 6.1 Hz, H-2a), 2.30 (1H, m, H-14b), 2.08 (3H, d, *J* = 1.0 Hz, H-27), 2.02 (2H, m, H-11), 1.73 (1H, d, *J* = 6.1 Hz, 7-OH), 1.69 (1H, m, H-8), 1.66 (3H, s, H-26), **XXX (H-$9_\alpha$, H-$9_\beta$, H-$10_\alpha$, H-$10_\beta$),** 1.21 (3H, d, *J*= 7.0 Hz, H-22), 1.16 (3H, d, *J*= 6.9 Hz, H-24), 0.94 (3H, d, *J*= 6.9 Hz,H-$25_\alpha$), 0.91 (3H, d, *J* = 6.4 Hz, H-$25_\beta$); **EIMS *m/z* 519 $[M]^+$ XXX; HREIMS *m/z* 519.XXX (calcd. for $C_{29}H_{45}NO_5S$, 519.XXX).**

**Epothilone K (36):** colorless amorphous solid; $[\alpha]^{22}_D$ **-7 (*c* 0.08, MeOH); UV (MeOH) $\lambda_{max}$ nm ($\epsilon$) 212 (16700), 248 (12500);** IR(KBr) $\nu_{max}$ 3431, 2963, 2927, 2856, 1731, 1712, 1262, 1093, 1021, 802 $cm^{-1}$; $^1H$ NMR ($CDCl_3$, 300 MHz) $\delta$ 6.95 (1H, s, H-19), 6.51 (1H, bs, H-17), 5.49 (3H, m, H-15, H-13, and H-12), 4.04 (1H, dddd, *J*= 7.9, 7.6, 6.9, 3.3 Hz, H-3), 3.36 (1H, dq, *J*= 6.9, 6.8 Hz, H-6), 2.83 (1H, d, *J*= 7.6 Hz, 3-OH), 2.75 (1H, ddd, *J*= 16.1, 6.6, 3.4 Hz, H-14a), 2.74 (1H, dd, *J* = 15.3, 3.3 Hz, H-2a), 2.71 (3H, s, H-21), 2.58 (2H, m, H-14b and H-8), 2.50 (1H, dd, *J* = 15.3, 7.9 Hz, H-2b), 2.29 (1H, m, H-11a), 2.10 (1H, m, H-11b), 2.09 (3H, d, *J*= 0.7 Hz, H-27), 1.78 (1H, m, H-9a), 1.65 (1H, m, H-10a), 1.48 (1H, m, H-10b), 1.18 (1H, m, H-9b), 1.15 (3H, d, *J* = 6.8 Hz, H-22), 1.03 (3H, d, *J* = 6.5 Hz, H-25); EIMS *m/z* 405 $[M]^+$ (38), 317 (12), 260 (9), 232 (10), 204 (14), 190 (16), 168 (100), 164 (30), 151 (28); **HREIMS *m/z* 405.XXX (calcd. for $C_{26}H_{39}NO_5S$, 405.XXX).**

**(37):** colorless amorphous solid; **$[\alpha]^{22}_D$ -27.5 (*c* 0.4, MeOH); UV (MeOH) $\lambda_{max}$ nm ($\epsilon$)** 211 (16100), 247 (12100); IR (KBr) $\nu_{max}$ 3431, 2967, 2929, 2875, 1704, 1462, 1381, 1010 $cm^{-1}$; $^1H$ NMR ($CDCl_3$, 400 MHz) $\delta$ 6.94 (1H, s, H-19), 6.55 (1H, bs, H-17), 5.56 (1H, dtt, *J* = 10.8, 7.3, 1.4 Hz, H-12), 5.39 (1H, dtt, *J* = 10.8, 7.3, 1.4 Hz, H-13), 4.17 (1H, t, *J* = 6.6 Hz. H-15), 3.50 (1H, ddd, *J* = 8.7, 2.6, 2.6 Hz, H-7), 3.10 (1H, d, *J* = 2.6, 7-OH), 2.90 (1H, dq, *J* = 2.6, 7.2 Hz, H-6), 2.77 (1H, sep, *J* = 6.9 Hz, H-4), 2.70 (3H, s, H-21), 2.40 (2H, m, H-14), 2.07 (2H, m, H-11), 2.04 (3H, d, *J* = 1.1 Hz, H-27), 1.78 (1H, bs, 15-OH), 1.74 (1H, m, H-9a), 1.50 (1H, m, H-8), 1.46 (1H, m, H-10a), 1.27 (1H, m, H-10b), 1.11 (1H, m, H-9b), 1.094 (3H, d, *J* = 6.9 Hz, H-23), 1.089 (3H, d, *J* = 6.9 Hz, H-22), 1.08 (3H, d, *J* = 7.2 Hz, H-24), 0.82 (3H, d, *J* = 6.7 Hz, H-25); $^{13}C$ NMR ($CDCl_3$, 100 MHz) $\delta$ 220.5 (s, C-5), 164.6 (s, C-20), 152.9 (s, C-18), 141.5 (s, C-16), 133.4 (d, C-12), 125.0 (d, C-13), 119.2 (d, C-17), 115.6 (d, C-19), 77.2 (d, C-15), 74.9 (d, C-7), 44.9 (d, C-6), 40.0 (d, C-4), 35.5 (d, C-8), 33.5 (t, C-14), 32.3 (t, C-9), 27.9 (t, C-11), 26.9 (t, C-10), 19.2 (q, C-21), 18.6 (q, C-23), 18.1 (q, C-22), 15.6 (q, C-25), 14.4 (q, C-27), 9.3 (q, C-24); EIMS *m/z* 407 $[M]^+$ (0.1), 204 (0.8), 168 (100), 140 (3.4); **HREIMS *m/z* 407.XXX (calcd. for $C_{23}H_{37}NO_3S$, 407.XXX).**

**(38):** colorless amorphous solid; $[\alpha]^{22}_D$ +25.0 (*c* 0.5, MeOH); UV (MeOH) $\lambda_{max}$ nm ($\epsilon$) 212 (17700), 247 (13400); IR (KBr) $\nu_{max}$ 3427, 2971, 2933, 2878, 2858, 1709, 1457, 1377, 1186, 1023 $cm^{-1}$; $^1H$ NMR ($CDCl_3$, 300 MHz) $\delta$ 6.95 (1H, s, H-19), 6.55 (1H, bs, H-17), 5.52 (1H, dtt, *J* = 10.9, 7.2, 1.4 Hz, H-12), 5.39 (1H, dtt, *J* = 10.9, 7.1, 1.2 Hz, H-13), 4.18 (1H, ddt, *J* = 3.4, 0.4, 6.7 Hz, H-15), 2.71 (3H, s, H-21), 2.51 (1H, bq, *J* = 6.8 Hz, H-8), 2.48 (1H, dq, *J* = 17.7, 7.4 Hz, H-6a), 2.41 (1H, dq, *J* = 17.7, 7.2 Hz, H-6b), 2.39 (2H, ddd, *J* = 7.1, 6.7, 1.4 Hz, H-14), 2.06 (2H, ddt, 7.2, 1.2, 7.0 Hz, H-11), 2.05 (3H, d, *J* = 1.4 Hz, H-27), 1.81 (1H, d, *J* = 3.4 Hz, 15-OH), 1.66 (1H, m, H-9a), 1.32 (1H, m, H-9b), 1.31 (2H, m, H-10), 1.06 (3H, d, *J* = 6.9 Hz, H-25), 1.04 (3H, dd, *J*= 7.4, 7.2 Hz, H-24); $^{13}C$ NMR ($CDCl_3$, 75 MHz) $\delta$ 215.3 (s, C-7), 164.6 (s, C-20), 152.9 (s, C-18), 141.5 (s, C-16), 132.7 (d, C-12), 125.3 (d, C-13), 119.2 (d, C-17), 115.6 (d, C-19), 77.2 (d, C-15), 46.0 (d, C-8), 34.3 (t, C-14), 33.5 (t, C-6), 32.7 (t, C-9), 27.5 (t, C-11), 27.3 (t, C-10), 19.2 (q, C-21), 16.5 (q, C-25), 14.4 (q, C-27), 7.8 (q, C-24); EIMS *m/z* 335 $[M]^+$ (2), 317 (4), 170 (27), 169 (67), 168 (100), 140 (20); HREIMS *m/z* 335.1912 (calcd. for $C_{19}H_{29}NO_2S$, 335.1919).

**(39):** colorless amorphous solid; **$[\alpha]^{22}_D$ +26.4 (*c* 0.27, MeOH); UV (MeOH) $\lambda_{max}$ nm ($\epsilon$) 203 (19100), 244 (12500);** IR (KBr) $\nu_{max}$ 3430, 2970, 2934, 2877, 1710, 1458, 1377, 1184 $cm^{-1}$; $^1H$NMR ($CDCl_3$, 400 MHz) $\delta$ 6.94 (1H, s, H-19), 6.55 (1H, bs, H-17), 5.17 (1H, t, *J*= 7.3 Hz, H-13), 4.13 (1H, m, H-15), 2.70 (3H, s, H-21), 2.51 (1H, bq, *J*= 6.8 Hz, H-8), 2.47 (1H, dq, *J* = 17.7, 7.2 Hz, H-6a), 2.41 (1H, dq, *J* = 17.7. 7.2 Hz, H-6b), 2.33 (2H, bdd, *J* = 7.3, 6.8 Hz, H-14), 2.05 (3H, d, *J*= 1.2 Hz, H-27), 2.03 (2H, m, H-11), 1.71 (1H, d, *J*= 3.2 Hz, 15-OH), 1.69 (3H, d, *J*= 1.3 Hz, H-26), 1.62 (1H, in, H-9a), 1.32 (3H, m, H-10 and H-9b), 1.06 (3H, d, *J* = 6.9 Hz, H-25), 1.03 (3H, t, *J*= 7.2 Hz, H-24); EIMS *m/z* 349 $[M]^+$ (0.7), 331 (1.7), 168 (100), 140 (5.1); **HREIMS *m/z* 349.XXX (calcd. for $C_{20}H_{31}NO_2S$, 349.XXX).**

Tab 1.

| Aktivität von Epothilonen und Verbindungen (1) bis (39) gegen Maus-Fibroblasten (L929, IC50 /ng/ml/) | | | | | |
|---|---|---|---|---|---|
| Struktur-typ | Epothilone | | | | |
| | $A_Y$ | $B_Y$ | $C_Y$ | $D_Y$ | trans $C_Y$ |
| Ausgangsepothilon | (**1**) 4 | (**2**) 1-2 | (**14**) 50-100 | (**15**) 20 | - |
| 21-Hydroxy (E&F) | (**3**) 10 | (**4**) 1.5 | - | - | - |
| Oxazoles (G&H) | (**10**) 6 | (**11**) 1 | (**12**) 120 | (**13**) 11 | - |

Tab 1. (fortgesetzt)

| Aktivität von Epothilonen und Verbindungen (1) bis (39) gegen Maus-Fibroblasten (L929, IC50 /ng/ml/) | | | | | |
|---|---|---|---|---|---|
| Struktur-typ | Epothilone | | | | |
| | $A_Y$ | $B_Y$ | $C_Y$ | $D_Y$ | trans $C_Y$ |
| Ausgangsepothilon | (**1**) 4 | (**2**) 1-2 | (**14**) 50-100 | (**15**) 20 | - |
| *(R)*-4-Desmethyl ($X_1$) | (**5**) 20 | - | (**16**) 200 | (**17**) 20 | (**28**) 400 |
| *(S)*-4-Desmethyl ($X_2$) | (**6**) 7 | - | (**18**) 25-30 | (**19**) 12 | (**29**) 80 |
| 6-Desmethyl ($X_3$) | - | - | (**20**) 1500 | - | - |
| 8-Desmethyl ($X_4$) | - | - | (**21**) 800 | - | - |
| 8,9-Dehydro ($X_5$) | - | - | (**22**) 1500 | (**23**) 200 | - |
| 10,11-Dehydro ($X_6$) | - | - | (**24**) 120 | - | - |
| 14-Hydroxy ($X_7$) | - | - | (**25**) | - | - |
| 16-Desmethyl ($X_8$) | (**7**) 20 | - | (**26**) 250 | - | - |
| 27-Hydroxy ($X_9$) | (**8**) 100 | - | (**27**) 200 | - | - |
| 21-Methyl ($X_{10}$) | - | (**9**) 1.5 | - | - | - |
| Verbindung | - | - | (**36**) 180 | | |
| Verbindung | - | - | (**37**) 50 | - | - |
| Verbindung | - | - | (**38**) 2000 | (**39**) 500 | - |

**Patentansprüche**

1. Epothilon der Formel

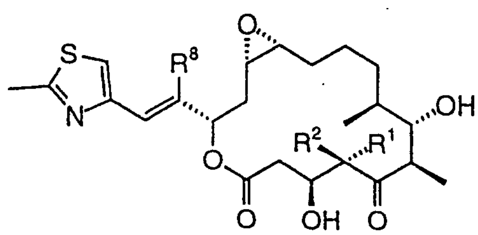

Epothilone $A_9$ (8) $R^1 = CH_2OH$; $R^2, R^8 = Me$

2. Epothilon der Formel

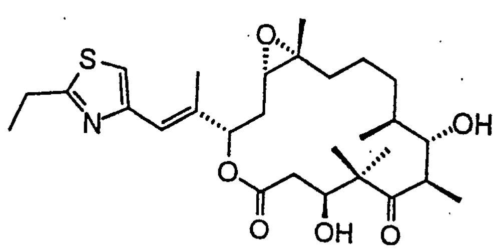

Epothilone $B_{10}$ (9)

3. Epothilon der Formel

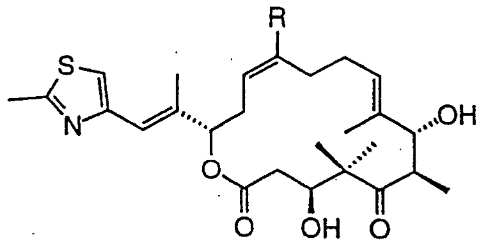

Epothilone $C_5$ **(22)** R = H

Epothilone $D_5$ **(23)** R = Me

oder

**4.** Epothilon der Formel

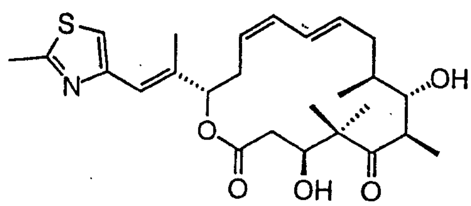

Epothilone $C_6$ **(24)**

**5.** Epothilon der Formel

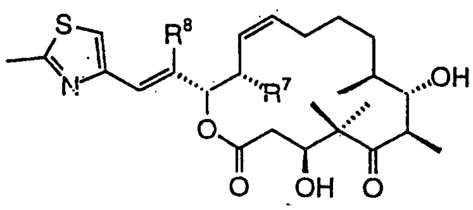

Epothilone $C_9$ **(27)** $R^8 = CH_2OH$; $R^7 = H$

**6.** Epothilon der Formel

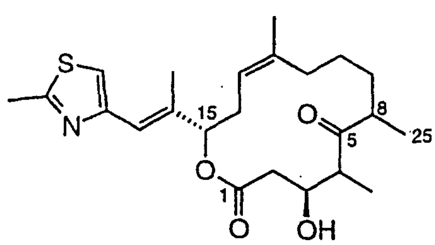

Epothilone K **(36)**

**7.** Verbindung der Formel

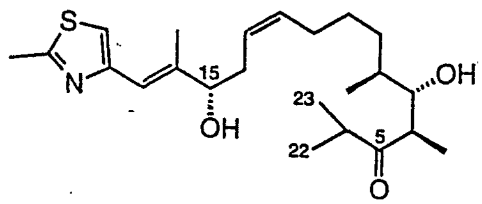

(37)

Fig. 1

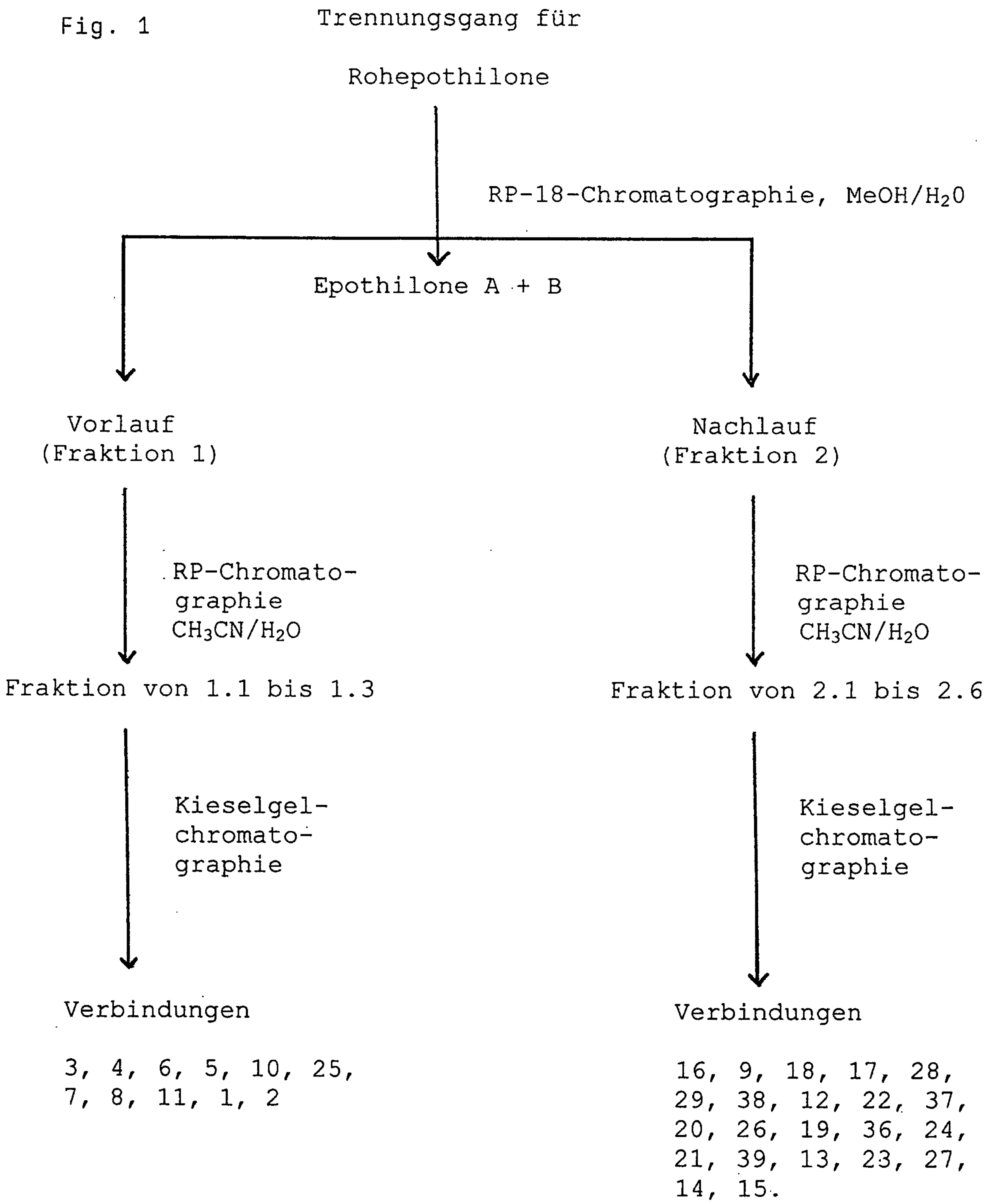
Trennungsgang für
Rohepothilone
RP-18-Chromatographie, MeOH/$H_2O$
Epothilone A + B
Vorlauf
(Fraktion 1)
Nachlauf
(Fraktion 2)
RP-Chromatographie $CH_3CN/H_2O$
RP-Chromatographie $CH_3CN/H_2O$
Fraktion von 1.1 bis 1.3
Fraktion von 2.1 bis 2.6
Kieselgelchromatographie
Kieselgelchromatographie
Verbindungen
3, 4, 6, 5, 10, 25, 7, 8, 11, 1, 2
Verbindungen
16, 9, 18, 17, 28, 29, 38, 12, 22, 37, 20, 26, 19, 36, 24, 21, 39, 13, 23, 27, 14, 15.

Fig. 2

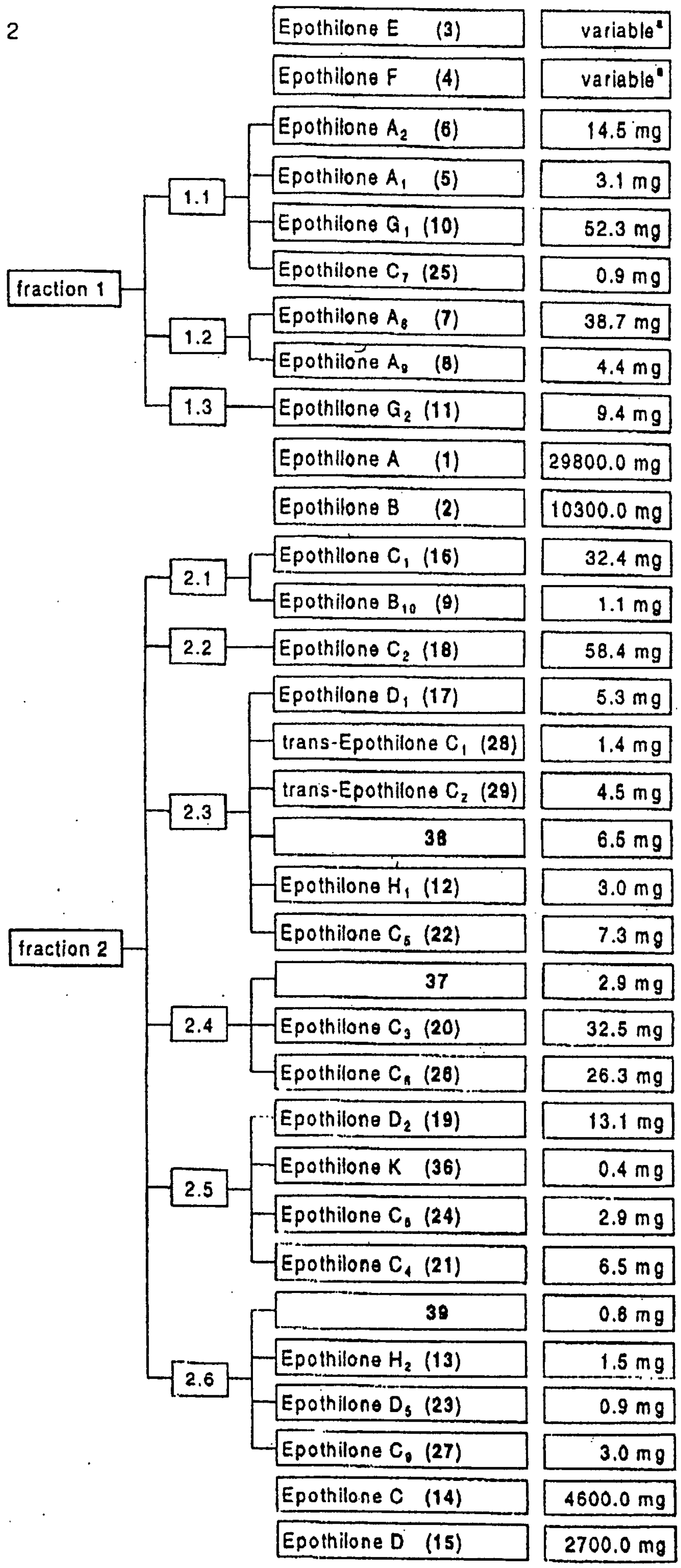

Epothilone E (3) variable[a]
Epothilone F (4) variable[a]
fraction 1
1.1
Epothilone $A_2$ (6) 14.5 mg
Epothilone $A_1$ (5) 3.1 mg
Epothilone $G_1$ (10) 52.3 mg
Epothilone $C_7$ (25) 0.9 mg
1.2
Epothilone $A_8$ (7) 38.7 mg
Epothilone $A_9$ (8) 4.4 mg
1.3
Epothilone $G_2$ (11) 9.4 mg
Epothilone A (1) 29800.0 mg
Epothilone B (2) 10300.0 mg
fraction 2
2.1
Epothilone $C_1$ (16) 32.4 mg
Epothilone $B_{10}$ (9) 1.1 mg
2.2
Epothilone $C_2$ (18) 58.4 mg
2.3
Epothilone $D_1$ (17) 5.3 mg
trans-Epothilone $C_1$ (28) 1.4 mg
trans-Epothilone $C_2$ (29) 4.5 mg
38 6.5 mg
Epothilone $H_1$ (12) 3.0 mg
Epothilone $C_5$ (22) 7.3 mg
2.4
37 2.9 mg
Epothilone $C_3$ (20) 32.5 mg
Epothilone $C_8$ (26) 26.3 mg
2.5
Epothilone $D_2$ (19) 13.1 mg
Epothilone K (36) 0.4 mg
Epothilone $C_6$ (24) 2.9 mg
Epothilone $C_4$ (21) 6.5 mg
2.6
39 0.8 mg
Epothilone $H_2$ (13) 1.5 mg
Epothilone $D_5$ (23) 0.9 mg
Epothilone $C_9$ (27) 3.0 mg
Epothilone C (14) 4600.0 mg
Epothilone D (15) 2700.0 mg

Europäisches Patentamt

# EUROPÄISCHER RECHERCHENBERICHT

Nummer der Anmeldung
EP 02 02 2332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A | WO 98 08849 A (NOVARTIS AKTIENGESELLSCHAFT ;BAUER ARMIN (DE); CORDES MARTIN (DE);) 5. März 1998 (1998-03-05) * Zusammenfassung; Ansprüche; Beispiele * --- | 1-7 | C07D493/04 C07D417/06 C07D413/06 C07D277/24 //(C07D493/04, 313:00,303:00) |
| A | WO 98 22461 A (BIOTECHNOLOG FORSCHUNG GMBH ;GERTH KLAUS (DE); HOEFLE GERHARD (DE)) 28. Mai 1998 (1998-05-28) * Zusammenfassung; Ansprüche; Beispiele * --- | 1-7 | |
| A | WO 97 19086 A (BIOTECHNOLOG FORSCHUNG GMBH ;KIFFE MICHAEL (DE); HOEFLE GERHARD (D) 29. Mai 1997 (1997-05-29) * Zusammenfassung; Ansprüche; Beispiele * ----- | 1-7 | |

RECHERCHIERTE SACHGEBIETE (Int.Cl.7)
C07D

Der vorliegende Recherchenbericht wurde für alle Patentansprüche erstellt

| Recherchenort | Abschlußdatum der Recherche | Prüfer |
|---|---|---|
| DEN HAAG | 21. November 2002 | Chouly, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 02 02 2332

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-11-2002

| Im Recherchenbericht angeführtes Patentdokument | Datum der Veröffentlichung | Mitglied(er) der Patentfamilie | Datum der Veröffentlichung |
|---|---|---|---|
| WO 9808849 A | 05-03-1998 | DE 19636343 C1 | 23-10-1997 |
| | | DE 19645361 A1 | 30-04-1998 |
| | | DE 19645362 A1 | 30-04-1998 |
| | | AU 716610 B2 | 02-03-2000 |
| | | AU 2149397 A | 19-03-1998 |
| | | WO 9808849 A1 | 05-03-1998 |
| | | EP 0923583 A1 | 23-06-1999 |
| | | JP 2001500851 T | 23-01-2001 |
| | | NZ 334821 A | 22-12-2000 |
| | | US 6043372 A | 28-03-2000 |
| | | US 6156905 A | 05-12-2000 |
| | | US 5969145 A | 19-10-1999 |
| WO 9822461 A | 28-05-1998 | AU 753546 B2 | 24-10-2002 |
| | | AU 5483798 A | 10-06-1998 |
| | | BR 9713363 A | 25-01-2000 |
| | | CN 1237970 A | 08-12-1999 |
| | | CZ 9901750 A3 | 15-09-1999 |
| | | WO 9822461 A1 | 28-05-1998 |
| | | EP 0941227 A1 | 15-09-1999 |
| | | HU 0000497 A2 | 28-06-2000 |
| | | IL 129558 A | 31-10-2001 |
| | | JP 2001504474 T | 03-04-2001 |
| | | NO 992338 A | 14-05-1999 |
| | | NZ 335383 A | 27-10-2000 |
| | | PL 333435 A1 | 06-12-1999 |
| | | TW 408119 B | 11-10-2000 |
| | | ZA 9710384 A | 18-05-1999 |
| WO 9719086 A | 29-05-1997 | DE 19542986 A1 | 22-05-1997 |
| | | DE 19639456 A1 | 26-03-1998 |
| | | AT 218556 T | 15-06-2002 |
| | | DE 59609305 D1 | 11-07-2002 |
| | | DK 903348 T3 | 16-09-2002 |
| | | WO 9719086 A1 | 29-05-1997 |
| | | EP 1186606 A1 | 13-03-2002 |
| | | EP 0873341 A1 | 28-10-1998 |
| | | EP 0903348 A1 | 24-03-1999 |
| | | JP 2000500757 T | 25-01-2000 |
| | | US 6288237 B1 | 11-09-2001 |
| | | US 2001034452 A1 | 25-10-2001 |

EPO FORM P0461

Für nähere Einzelheiten zu diesem Anhang : siehe Amtsblatt des Europäischen Patentamts, Nr.12/82